# EUROPÄISCHE PATENTANMELDUNG

(11) **EP 1 361 438 A1**
(43) Veröffentlichungstag der Anmeldung: **12.11.2003**
(21) Anmeldenummer: 02010555.7
(22) Anmeldetag: 10.05.2002
(51) Int. Cl.: G01N 33/68

(54) **Festphasenassistierte spektroskopische und spektrometrische Analyse komplexer Peptidgemische**

(71) Anmelder: Proteome Factory AG, 14167 Berlin (DE)
(72) Erfinder: Scheler, Christian, Dr., 14167 Berlin (DE); Essmann, Frank,Dr., 10439 Berlin (DE); Thies, Sascha, 12099 Berlin (DE)
(74) Vertreter: Helbing, Jörg, Dr. Dipl.-Chem.

(57) **Zusammenfassung**

Die vorliegende Anmeldung betrifft ein Verfahren zur Analyse komplexer Mischungen von Peptiden. Das problem ist die Bereitstellung eines Verfahrens zur Analyse komplexer Mischungen von Peptiden mit einer deutlichen Reduktion der zu analysierenden Komponenten bei nur geringem Verlust des Informationsgehaltes.

Diese Aufgabe wird gelöst durch eine gezielte Selektion der Peptidfragmente durch Kopplungs- und Entkopplungsreaktionen an einen festen Träger, sowie Fragmentierung. Es gibt verschiedene Ausführungsformen:
Eine erste Ausführungsform nützt die vorteilhafte Eigenschaft gekoppelter N-terminaler Peptiden aus, daß selektive Freisetzung vom Trägermaterial möglich ist, wenn pDITC als Linker verwendet wird, wohingegen Peptide welche über Aminosäureketten mit NH₂- Funktionalität gekoppelt sind nicht freigesetzt werden.
Eine zweite Ausführungsform verwendet Blockierung der N-Termini von Peptiden die danach gespaltet werden. Danach findet Kopplung an den festen Träger statt und werden nur Peptide mit einem freien N-Terminus immobilisiert, wohingegen die blockierten N-terminalen Peptide mittels eines Waschschrittes isoliert werden können. Optional findet eine Markierung der Peptide statt. Danach kann die Mischung mit Massenpektrometrie- oder Fluoreszenzpektroskopie analysiert werden.
Auch wird die Immobilisierung von Peptiden nach Spaltung mit CNBr via Homoserinlacton über die Amingruppe auf den festen Träger vorgeschlagen.

## Beschreibung

### Kurze Zusammenfassung der Erfindung

Die vorliegende Erfindung betrifft ein Verfahren und einen Kit zur Analyse komplexer Mischungen von Biopolymeren aus einer oder mehreren Proben.

### Hintergrund der Erfindung

Während der Suche nach molekularen Ursachen für diverse Krankheiten hat die Molekularbiologie/DNA-Analyse einen Punkt erreicht, an dem es möglich ist, die gesamte genomische Information sogar für komplexe Organismen auszulesen. Im Einvernehmen hiermit ist der Abschluss des Humangenom-Projektes in greifbare Nähe gerückt und die Sequenzierung der Genome anderer Organismen ist schon abgeschlossen. Des weiteren kann die mRNA Expression für eine immense Anzahl an Genen simultan quantifiziert werden. Mittels der DNA-Array Technologie kann sogar der Einfluß von Umweltbedingungen auf das Expressionsniveau von Genen ermittelt werden. Das heißt, dass qualitative und quantitative Information über Gene und Genexpression in Organismen und Geweben zumindest theoretisch gewonnen werden kann.

Diese immense Menge an zugänglichen und vorhandenen Daten verlangt nach einem intelligenten und leistungsstarken Management. Aus diesem Grund war die Einführung schneller und leistungsfähiger Computer sowie die Entwicklung intelligenter Software eine Voraussetzung für den Umgang mit derart enormen Datenmengen. In der Zwischenzeit ist der Umgang mit enormen Datenmengen im Routinebetrieb implementiert, sowohl hinsichtlich der Strukturaufklärung, biochemischer Wechselwirkungen, Regulation und Funktion von Genen/Proteinen. Unglücklicherweise liegt es trotz allem in der Natur genetischer Information, dass sie keinerlei Rückschlüsse auf Regulationsmechanismen oder das Expressionsniveau von Proteinen erlaubt.

Dem allgemein akzeptierten Dogma der Biologie folgend, sind Proteine die aktiven Komponenten biologischer Systeme wohingegen die DNA lediglich ein Speichermedium für die Information zur Herstellung von Proteinen darstellt. Bestimme Typen von RNA fungieren als Bindeglied zwischen dem Informationsspeicher DNA und den funktionierenden Komponenten, den Proteinen, sei es durch Übersetzung der in der DNA enthaltenen Information in eine umsetzbare Form oder als Träger kleinster Einheiten von Information, gekoppelt an die Grundbestandteile von Proteinen (Aminosäuren).

Es ist kein biologisches System bekannt in dem diese Richtung des Informationsflusses umgekehrt würde. Aus diesem Grund ist die Analytmenge, die proteinanalytischen Verfahren zur Verfügung steht, im allgemeinen stark limitiert. Die z.Zt. eingesetzte Methodik zur Identifizierung und Quantifizierung aller unter bestimmten Umweltbedingungen in einem System repräsentierten Proteine muß daher zwingend extrem sensitiv sein. Weiterhin können Proteine posttranslational modifiziert werden, wodurch z.B. ihre Halbwertzeit, biologische Funktion und/oder Aktivität beeinflusst werden können. Diese posttranslationalen Modifikationen sind z.B. Phosphorylierung, Glykosylierung, Farnesylierung, die Bindung von Nukleotiden und Metallionen, um nur einen Bruchteil der Möglichkeiten zu nennen. Alle diese Informationen sind allein aufgrund genetischer Information nicht zugänglich.

Die erwähnte komplette Beschreibung des gesamten Proteingehaltes eines biologischen Systems unter definierten Bedingungen inklusive Expressionsniveau, Modifikationen und Identität der Proteine nennt sich Proteom. Die Proteomanalyse ist der kommende Meilenstein der Lebenswissenschaften nach der kompletten Sequenzierung des humanen Genoms und aufgrund der o.g. Gründe und Limitationen ein unvergleichlich komplexeres und umfangreicheres Ziel. Aus diesen Gründen erscheint es unerlässlich, die Komplexität der zu analysierenden Proben auf ein Maß zu reduzieren, das mit den verfügbaren Mitteln handhabbar ist, ohne die enthaltene Information zu reduzieren.

Wie oben beschrieben existiert keine proteinanalytische Methodik mit einer der DNA/RNA-Analytik vergleichbaren Empfindlichkeit und/oder einem vergleichbaren Durchsatz, da Proteine nicht *in vitro* amplifiziert werden können. Zur Zeit finden zwei grundsätzlich unterschiedliche Technologien zur Analyse komplexer biologischer Gemische Anwendung. Dies sind zum einen die zweidimensionale Gelelektrophorese und zum anderen die in den Anfängen befindliche mehrdimensionale Flüssigchromatographie. Ein drittes Verfahren, das sogenannte ICAT-Verfahren, bedient sich spezifischer chemischer Komponenten, die eine (nicht-)radioaktive Affinitätsmarkierung in Biopolymere einführen (WO 00/11208).

Die zweidimensionale Gelelektrophorese (2DE) ist die verbreitetste Technologie zur Proteomanalyse. Das komplexe Proteingemisch wird in zwei Dimensionen in homogenen Polymermatrix getrennt. Hierbei wird das komplexe Gemisch der Biopolymere (Proteine) zunächst basierend auf dem isoelektrischen Punkt der Komponenten getrennt. Die so in der Matrix getrennten Proteine werden anschließend in einer analogen Matrix nach ihrem scheinbaren Molekulargewicht getrennt. Die folgende Färbung resultiert nun in einem spezifischen Punktmuster, in dem jeder Punkt idealerweise einem Protein entspricht.

Zur zweidimensionalen gelelektrophoretischen Trennung von Proteinen werden zur Zeit zwei unterschiedlicher Verfahren eingesetzt. Dies ist die von J. Klose (Humangenetik 26(3), 231 - 243 (1975)) bzw. O'Farel publizierte NEPHGE (non-equilibrium pH gradient electrophoresis) und die von A. Görk et. al. (Elektrophoresis 9(1), 57 - 59 (1988)) entwickelte IPG-Technik. Die beiden Verfahren unterscheiden sich hauptsächlich in der Durchführung der ersten Dimension (isolelektrische Fokussierung). Hierbei wird ein pH-Gradient etabliert in dem die Proteine nach Anlegen eines elektrischen Feldes aufgrund ihrer Ladung zu dem Punkt wandern, an dem ihre Ladung Null ist. An diesem Punkt wirkt keine elektrische Kraft auf die Proteine und diese bleiben dort in der PAA Matrix. Der pH Gradient wird entweder durch mobile Ampholyte aufgebaut (NEPHGE) oder bei der Herstellung der Matrix in das Gel einpolymerisiert (IPG-Technik). Die Methode beschränkt sich aber nicht auf PAA-Matrices sondern ist mit anderen Materialien (z.B. Agarose) kompatibel. Weiterhin existieren Systeme die eine isolelektrische Fokussierung in Lösung erlauben.

Die Trennmatrix, welche die eindimensional getrennten Proteine enthält, wird nun auf eine weitere Matrix transferiert, in der die weitere Trennung (2. Dimension) vorgenommen wird. Diese besteht üblicherweise aus einer SDS-PAGE (Natriumdodecylsulfat-Polyacrylamid-Gelelektrophorese). Die Proteine werden hierdurch in der Matrix nach ihrem scheinbaren Molekulargewicht getrennt.

Der gesamten Prozedur schließt sich eine Färbung an, durch die die Proteine/Polymere sichtbar gemacht werden. Es ergibt sich ein probespezifisches Punktmuster, das einer detaillierten vergleichenden Analyse unterzogen wird.

Die Multi-Dimensionale Flüssigphasenchromatographie (MDLC) ist eine Methode, die noch nicht routinemäßig in der Analytik eingesetzt wird, und ihre Verbreitung bleibt weit hinter der der 2DE zurück. Sie weist allerdings diverse Vorteile gegenüber der 2DE auf. Die komplexen Protein/Biopolymer-Gemische werden auf der Grundlage spezifischer Interaktionen mit der Oberfläche des Trennmaterials voneinander getrennt. Je nach individueller Zusammensetzung zeigen die Biopolymere eine spezifische Retentionszeit.

Die separierten Biopolymere müssen nach erfolgter Trennung identifiziert werden (Protein-Identifizierung). Im Fall der 2DE geht man hierbei folgendermaßen vor. Nach der Färbung werden die interessierenden Spots aus der Matrix ausgeschnitten und mittels enzymatischer oder chemischer Reaktion in kleinere Fragmente zerlegt. Die Fragmente können aus der Matrix diffundieren und werden der anschließenden massenspektrometrischen Analyse zugeführt. Hierbei werden die Massen der erzeugten Fragmente ermittelt, wodurch sich im Zusammenhang mit anderen bekannten Daten zu dem jeweiligen Analyten eine Identifizierung (eindeutige Zuordnung zu einem Datensatz einer Proteindatenbank) erreichen lässt. Erfolgt die Analyse in einem MS/MS-fähigen Gerät, so kann eine Fragmentierung eines spezifischen Fragmentes durchgeführt und so dessen Zusammensetzung genauer ermittelt werden. Durch Kenntnis über die Zusammensetzung eines oder mehrerer vorhandener Fragmente ist es möglich, die Identifizierung des Ausgangsanalyten mit größerer Wahrscheinlichkeit zu erreichen. Das sequenzielle Vorgehen bei dieser Analyse ist der limitierende Schritt bei der Identifizierung der Proteine. Massenspektrometer benötigen zur Analyse mitunter nur wenige fmol eines Analyten. Da nun aber die größten Verluste während der Vorbereitung des Biopolymers für die MS-Analyse auftreten, muß weit mehr Ausgangsmaterial zur Verfügung stehen.

In jüngster Vergangenheit hat die Entwicklung von Methoden und Instrumenten zur automatischen und datenabhängigen massenspektrometrischen Analyse im Zusammenhang mit der Mikrokapillarelektrophorese die Sensitivität und Geschwindigkeit gel-separierter Proteine ebenso wie die Analyse zuvor fragmentierter komplexer Biopolymergemische maßgeblich verbessert. In dieser Hinsicht hat die Identifikation von Proteinen deutliche Fortschritte gemacht, wohingegen die (relative) Quantifizierung nach wie vor äußerst problematisch ist.

Der zur Quantifizierung der Proteine zur Verfügung stehende dynamische Bereich der verschiedensten Färbe- und Detektionsmethoden deckt nicht die erforderlichen Größenordnungen ab. Proteine können in einer einzigen Zelle in Kopienzahlen von 1 bis zu Millionen vorhanden sein, was die Problematik verdeutlicht. In der 2DE kommen im allgemeinen folgende Färbemethoden zum Einsatz:

| Methode | Detektionsgrenze / ng |
|---|---|
| Silber mit Glutardialdehyd¹ | 10 |
| Silber² | 1 |
| Zink/Imidazol | 100 |
| KCI | 100 |
| Coomassie R250 | 100 |
| kolloidales Coomassie G250 | 30 |
| Fluoreszenz | 10 |

| | |
|---|---|
| ¹ keine weitere Verarbeitung (Identifizierung) möglich | |
| ² geeignet zur anschließenden massenspektrometrischen Analyse | |

Im relevanten Technologiefeld existieren sehr wenige Patente/Patentanmeldungen:

In WO 00/11208 wird das ICAT-Verfahren beschrieben, womit ein oder mehrere Proteine oder Proteinfunktionen in einer oder mehreren Proben identifiziert werden können, so dass eine qualitative und quantitative Analyse von Expressionsprofilen möglich ist. Hierzu wird ein Markierungsreagenz eingesetzt, welches für jede Probe verschieden isotopenmarkiert ist, wodurch eine quantitative Bestimmung von relativen Mengen von Proteinen in verschiedenen Proben möglich ist. Des weiteren weist das Markierungsreagenz eine Affinitätsmarkierung, einen Linker und eine proteinreaktive Gruppe auf, welche entweder mit einer funktionellen Gruppe eines Proteins oder als Substrat eines Enzyms reagiert. Jede Probe wird mit einem Markierungsreagenz versetzt, und affinitätsmarkierte Proteine oder Enzymprodukte werden hergestellt, die dann mit Abfangreagenzien, die selektiv eine Affinitätsmarkierung binden, abgefangen werden. Nach Freisetzung der affinitätsmarkierten Komponenten erfolgt die Detektion und Identifikation der freigesetzten affinitätsmarkierten Komponenten mittels Massenspektrometrie. Ein Nachteil dieses Verfahrens ist jedoch seine Beschränkung auf Proteine und die Isotopenmarkierung als einzige Markierung, die eine Zuordnung des jeweiligen Proteins zur Ausgangsprobe sicherstellt und eine quantitative Bestimmung relativer Mengen von Proteinen aus verschiedenen Proben ermöglicht. Ferner ist durch das in WO 00/11208 offenbarte Verfahren keine Sequenzierung d.h. die Bestimmung der Primärstruktur von Proteinen und auch keine Reduktion des Probenaufkommens durch die Zusammenführung gleichartiger Moleküle vor der massenspektrometrischen Untersuchung möglich.

EP-A-1106702 betrifft ein Hochdurchsatz-Screeningverfahren zum Auffinden von nichtkovalenten Wechselwirkungen zwischen einer oder mehreren Testverbindungen und Polynukleotiden, wobei sich die Polynukleotide in einem Gleichgewicht zwischen der einzelsträngigen und der doppelsträngigen Form befinden. Die aufgrund nichtkovalenter Wechselwirkungen entstehenden Komplexe aus Testverbindung und Polynukleotid in Lösung werden dann mittels ESI-MS (electrospray ionization mass spectrometry) untersucht. Somit beschränkt sich das Verfahren der EP-A-1106702 auf das Auffinden von Verbindungen, die mit Polynukleotiden wechselwirken, befasst sich aber nicht mit der Strukturaufklärung der Polynukleotide selbst und auch nicht anderer Biomoleküle. Schließlich ist auch keine Reduktion des Probenaufkommens vor der ESI-MS-Untersuchung vorgesehen. Ein Verfahren welches die Flüssigchromatographie mit ESI-MS (LC-ESI-MS Analyse) verbindet ist in US 6,139,734 offenbart, wobei die Trennung der zu testenden Verbindungen, insbesondere biologisch relevanter Verbindungen, mittels Hochleistungsflüssigkeitschromatographie (HPLC) unter Kontrolle der Fließgeschwindigkeit der mobilen Phase in der HPLC-Säule erfolgt. Das Verfahren der US 6,139,734 ermöglicht also ebenfalls keine Identifizierung von Biomolekülen und auch keine Reduktion des Probenaufkommens vor der ESI-MS-Untersuchung.

WO 02/29414 A2 beschreibt ein Verfahren, bei dem ein oder mehrere Biomoleküle (z.B. Proteine, Peptide) in einer oder mehrerer Proben unterschiedlich Massen markiert werden (mass tagging). Nach der unterschiedlichen Markierung werden die verschiedenen Proben vereinigt, ihre Bestandteile (Biomoleküle) z.B. durch Chromatographie separiert und die einzelnen Fraktionen im Massenspektrometer vermessen. Durch die Massemarkierung können die Biomoleküle quantifiziert und den einzelnen Proben zugeordnet werden.

WO 00/67017 beschreibt ein Verfahren zur *in-vivo* Isotopenmarkierung von Proteinen in biologischem Material. Eine Probenkultur wird mit einem (Nähr)medium inkubiert, das ein bestimmtes Isotop enthält . Eine andere Kultur wird mit einem anderem Isotop inkubiert. Beide Proben, die so unterschiedlich isotopenmarkiert sind, werden zusammen gegeben, die Protein extrahiert und durch chromatographische oder andere Verfahren fraktioniert. Die einzelnen Fraktionen können dann durch Massenspektrometrie analysiert und relativ quantifiziert werden. Durch die Isotopenmarkierung können die Biomoleküle ihrer Ursprungsprobe zugeordnet werden.

Darüber hinaus existieren die folgenden relevanten Publikationen:

M. B. Smolka et al., Anal. Biochem., 297(1):25-31 (2001) beschreibt die systematische Optimierung der ICAT-Methode unter Variation der Konzentrationen verschiedener Chemikalien (z.B. SDS, Harnstoff) und Reaktionsbedingungen (Dauer). Es wird eine spezifische und quantitative Markierung der Analyte demonstriert.

T. J. Griffin, J. Am. Soc. Mass Spectrom., 12(12):1238-46 (2001) beschreibt eine Verbesserung des ICAT-Verfahrens. Zur Steigerung der Effektivität werden erst die Peptide identifiziert, welche in unterschiedlicher Quantität in den zu vergleichenden Proben vorhanden sind. Nur die unterschiedlich repräsentierten Peptide werden einer detaillierten massenspektrometrischen Analyse (Fragmentierung und MS/MS) zugeführt. Vorgestellt wird in diesem Zusammenhang eine entsprechende Software, die die beschriebene Analyse automatisiert.

D. K. Han et al., Nat. Biotechnol. Oct; 19(10):946-51 (2001) beschreiben eine spezielle Anwendung der ICAT-Technologie. Den ersten Schritt stellt die Präparation der zu vergleichenden mikrosomalen Fraktionen da. Diese werden anschließend mit dem isotopenmarkierten Affinitäts-Tag versehen, vermischt und enzymatisch gespalten. Es schließt sich eine multidimensionale chomatographische Trennung der resultierenden Peptidgemische an. Die chromatographische Trennung muendet direkt in ein Tandem Massenspektrometer, das sowohl die Analyse der relativen Intensitäten zweier Peptidpeaks als auch der Ermittlung von Sequenzinformation oder dieselben gestattet. Die Autoren beschreiben die Anwendung der Methode zur Identifizierung und relativen Quantifizierung von 491 Proteinen in nativen und *in vitro* differenzierten HL-60 Zellen.

F. Turecek, J. Mass Spectrom., 37:1-14 (2002) gibt einen Überblick über Anwendungen der ICAT-Technik als auch der verwandten ACESIMS-Technik. Beide Methoden werden detailliert beschrieben und Beispiele für deren Anwendung zur Diagnose von beispielsweise GM1 oder Mucopolysaccharidosis Typ III (Sanfilippo Syndrom Typ A-d) gegeben.

In M. B. Goshe et al., Anal. Chem., 73(11):2578-86 (2001) wird die selektive Aufreinigung und Anreicherung O-Phosphorylierter Peptide beschrieben. Die Methode stellt ein leicht abgewandeltes Verfahren der ICAT-Methode dar. Nach der β-Elimination durch Hydroxid wurde ein funktionalisiertes Linker-Reagenz in deuterierter oder undeuterierter Form an die entstandene Doppelbindung addiert (Ethandithiol). Die hierdurch erreichte Funktionalisierung (Thiol-Gruppe) wurde ähnlich dem ICAT-Verfahren zur Kopplung an biotinyl-iodoacetamidyl-3,6-dioxaoctanediamine genutzt. Mit Hilfe des Biotins können nun die zuvor O-phosphorylierten Peptide selektiv affinitätsgereinigt werden. Die massenspektrometrische Analyse nach Trennung der Peptide erlaubt eine relative Quantifizierung anhand der Peakintensitäten ( Dm = 4 Da).

G. Cagney, A. Emili, Nature Biotech, 20(2): 163-170 (2002) beschreiben ein Verfahren zur Markierung c-terminaler Lysine in Peptiden. Die Methode beruht auf der tryptischen Spaltung von komplexen Proteingemischen, anschließender Markierung und Trennung mittels Kapillar-Electrophorese. Zur Detektion und Quantifizierung wird ein Electrospray-Tandem-Massenspektrometer eingesetzt.

In A. J. Forbes et al., Proteomics, 1(8):927-33 (2001) werden die Vorteile einer Lys-C Spaltung gegenüber der Verwendung ungespaltener Proteine zur Analyse in der FT Massenspektrometrie erläutert.

C. S. Spahr et al., Electrophoresis, 21(9):1635-50 (2000) schildern die selektive Affinitätsreingung cysteinhaltiger Peptide in einer Modellmischung aus Proteinen sowie in einem Modellsystem. Die Proteinprobe wird mittels kommerziell erhältlicher Reagenzien an vorhandenen Cysteinen biotinyliert und anschließend tryptisch gespalten. Die biotiynlierten Peptide werden mit Hilfe einer Streptavidin-Säule chromatoghraphisch abgetrennt. Die so abgetrennten Peptide werden anschließend durch Zugabe von DTT wieder freigesetzt. Es gehen sowohl die nicht-gebundenen als auch die gebundenen Peptide nach Alkylierung in die weitere massenspektrometrische Analyse. Diese wird mittels LC-MS/MS durchgeführt.

T. J. Griffin et al., Anal. Chem. Mar 1;73(5):978-86 (2001) beschreiben die Verwendung isotopenmarkierter Affinitäts-Tags in Kombination mit MALDI-QqTOF Massenspektrometrie zur quantitativen Analyse komplexer Proteingemische. Die Proteingemische werden hier initial markiert, enzymatisch gespalten und mittels einer multidimensionalen Chromatographie getrennt, bei der die Elution direkt auf das MALDI-Target erfolgt. Die massenspektrometrische Analyse besteht zum einen aus der Aufnahme eines Massenspektrums der Peptide zur Bestimmung der Intensitätsrelation und anschließender Fragmentierung zur Sequenzermittlung.

Aufgabe der vorliegenden Erfindung ist es daher, ein Verfahren bereitzustellen, wodurch komplexe Mischungen von Biopolymeren aus einer oder mehreren Proben fragmentiert, durch gezielte Kopplung und Entkopplung an feste Trägermaterialien selektioniert, probenspezifisch markiert und nach Vereinigung der Proben getrennt und analysiert (z.B. Quantifizierung und Identifizierung/Sequenzierung durch Massenspektrometrie) werden. Diese Erfindung soll zu geringen Verlusten des Gesamtinformationsgehaltes der Ausgangsbiopolymermischungen bei der Analyse, d.h. Identifizierung und Quantifizierung ihrer Bestandteile im Vergleich zu den oben aufgeführten Verfahren führen. Dazu dient eine gezielte Selektion der Biopolymer(fragment)e durch Kopplungs- und Entkopplungsreaktionen sowie Fragmentierung, die eine deutliche Reduktion der zu analysierenden Komponenten bewirkt, ohne den Informationsgehalt der Ausgangsproben signifikant zu verringern. Durch probenspezifische Fluoreszenzmarkierung der Biopolymer(fragment)e ist zusätzlich eine Vorselektion der durch Massenspektrometrie zu analysierenden Moleküle schon während der Trennschritte möglich, wodurch eine gezielte Reduktion der zu analysierenden Komponenten erreicht wird.

Überraschenderweise wurde nun gefunden, das insbesondere bei der differentiellen Analyse von Protein- und Peptidgemischen im Rahmen der Proteom- und Peptidomanalyse durch gezielte kovalente Kopplung und Entkopplung von Biopolymeren bzw. deren (chemischen bzw. enzymatischen) Spaltprodukten an feste Trägermaterialien eine deutliche Reduktion der zu analysierenden Komponenten bei nur geringem Verlust des Gesamtinformationsgehaltes der Ausgangsbiopolymermischungen erzielt werden kann (Ausführungsform A). Die differentielle Analyse von zwei oder mehr Biopolymergemischen wird durch probenspezifische Markierungen erreicht, die geringe, aber messtechnisch nachweisbare Unterschiede (Fluoreszenzfarbstoffe mit ähnlicher Struktur, Marker mit Masseunterschieden bspw. durch Isotopenmarkierung) aufweisen. Nach der Trennung der zusammengegebenen Biopolymergemische (Chromatographie, Elektrophorese etc.) in ihre Komponenten oder in Komponentengemische erfolgt die Analyse (Quantifizierung, Identifizierung, Charakterisierung) einzelner Komponenten vornehmlich durch Massenspektrometrie. Durch Einsatz von (Fluoreszenz-)Spektrometern ist nach der Gemischtrennung zusätzlich aufgrund von geeigneten Markierungen wie Fluoreszenzmarkierungen eine gezielte Vorselektion von Fraktionen möglich, in denen z. B. Intensitäts-, d. h. Mengenunterschiede von Komponenten des Gesamtgemisches vorliegen. Dies führt zu einer Reduktion des Zeit- und Kostenaufwandes in der massenspektrometrischen Analyse, da nur ausgewählte Fraktionen differentiell untersucht werden.

Als Lösungsansatz sei hier vor allem die gezielte Analyse ausschließlich Lysin-freier Peptid(fragment)e mit freiem Amino-Terminus (Ausführungsform B) genannt. Bei diesem Verfahren werden zuerst alle Proteine/Peptide durch chemische bzw. enzymatische Reaktionen in Fragmente zerlegt. Diese Fragmente werden über ihre N-terminalen Aminogruppen und, wenn vorhanden, über Aminogruppen aus Lysinen mittels Isothiocyant-Derivaten an feste Trägermaterialien gebunden. Anschließend erfolgt die Entkopplung von Biopolymeren (Fragmenten), die nur über ihre N-terminale Aminogruppe gebunden wurden, da nur in diesem Fall die Bildung von Anilinothiazolidinon möglich ist. Anschließend werden die Biopolymere des so freigesetzten Biopolymergemisches markiert, z.B. mit Fluoreszenzmarkern. Alle parallel behandelten und unterschiedlich markierten (z.B. unterschiedliche Massemarkierung, unterschiedliche Fluoreszenzmarkierung) Probengemische werden dann für die weitere differentielle Analyse zusammengegeben. Es folgt eine Trennung der Gemische und Analyse mittels spektroskopischer und/oder massenspektrometrischer Verfahren, um die Probenkomponenten zu quantifizieren sowie sie zu charakterisieren und zu identifizieren (u.a. Gewinnung von Sequenzinformationen durch MS/MS). Vorteilhaft bei diesem Verfahren ist, dass eine starke Verringerung der zu analysierenden Fragmente gegenüber dem gesamten Ausgangsspaltungsgemisch erreicht wird, ohne den Informationsgehalt bezüglich der Ausgangpolymergemische (Proteome) signifikant zu verringern.

In Ausführungsform C ist eine Variante erfunden worden, die eine selektive Analyse aller N-Termini von Proteinen und Peptiden aus Gemischen erlaubt. Dazu werden zuerst alle Lysine und N-terminalen Aminogruppen der Proteine/Peptide blockiert, bspw. mit Citraconsäureanhydrid. Anschließend erfolgt eine Spaltung der Proteine/Peptide durch chemische bzw. enzymatische Reaktionen in Fragmente. Im nächsten Schritt werden analog zu Ausführungsform B alle Aminogruppen-haltigen Polymere an feste Träger gebunden. Nur die ursprünglichen N-Termini der Proteine/Peptide werden nicht gebunden und können für eine weitere Markierung, Trennung/Analyse eluiert und gesammelt werden. Gebundene Peptide können zusätzlich sequentiell selektiv entkoppelt werden, indem eine Spaltung der Peptide an Methioninen durch CNBr-Behandlung erfolgt oder eine Entkopplung in Analogie zu Ausführungsform B durchgeführt wird. Wie oben beschrieben, folgen die weiteren Analysenschritte (Trennung und spektroskopische/spektrometrische Analyse zur Quantifizierung und Charakterisierung/Identifizierung), nachdem die verschiedenen Peptide der unterschiedlichen Ausgangsproben probenspezifisch markierten und zusammengegeben wurden.

Vorteil dieser Ausführungsform ist, dass pro Protein nur das N-terminale Peptid analysiert wird und somit die maximale Reduktion der Komplexität auf ein Peptid pro Protein erreicht wird. Dies ist mit keinem herkömmlichen Verfahren auch nur annähernd möglich.

### Zusammenfassung der Erfindung

Die vorliegende Erfindung betrifft daher ein Nachweisverfahren unter Einsatz von chemische und/oder biochemische Reagenzien, Separationsmethoden und spektrometrische sowie massenspektrometrische Methoden, die diese Reagenzien zur Analyse von Verbindungen und komplexe Mischungen von Verbindungen einsetzen. Es werden insbesondere Chemikalien und/oder biologische Verbindungen (z. B. Enzyme) genutzt, die mit Biopolymeren reagieren und diese fragmentieren. Zusätzlich kommen Verbindungen zum Einsatz, die eine selektive Kopplung und Entkopplung dieser Biopolymere an bzw. von Trägermaterialien ermöglichen.

Die Polymere oder Teile von Polymeren (im folgenden kurz Proteine/Peptide oder Verbindungen) werden unter Einsatz dieser Reagenzien kovalent an einen unlöslichen Träger gebunden und ein spezifischer Teil aller gebundenen Moleküle wird unter Einsatz spezifischer Reagenzien wieder von dem Träger freigesetzt (Lösen der Bindung). Die freigesetzten (sowie andere ungebundene) Moleküle können im weiteren an Moleküle gekoppelt werden, die eine Detektion, die nicht auf den Eigenschaften der freigesetzten Moleküle basiert, gewährleisten, wie z.B. fluoreszierende oder radioaktive Verbindungen (Markierung). Die freigesetzten Moleküle (z.B. Peptide) werden detailliert charakterisiert, insbesondere unter Einsatz massenspektrometrischer Methoden oder anderer Methoden, die eine Aussage über die molekulare oder atomare Zusammensetzung der Moleküle erlauben. Die beschriebene Methode kann zur qualitativen und quantitativen Analyse von Biopolymeren in komplexen Mischungen eingesetzt werden, um die Identität (Zusammensetzung) und die Menge von Biopolymeren in mindestens zwei unterschiedlichen Lösungen zu quantifizieren. Die Markierung der ungebundenen Verbindungen kann zu ihrer Detektion und Quantifizierung herangezogen werden. Die (mindestens zwei) komplexen Mischungen ungebundener Verbindungen können gemischt werden und die unterschiedliche Markierung erlaubt eine Zuordnung der untersuchten Verbindung zu einer der miteinander gemischten Lösungen. Die unterschiedliche Markierung erlaubt weiterhin eine Reduktion der zu analysierenden Verbindungen auf diejenigen, welche in unterschiedlicher Menge in unterschiedlichen Lösungen vorhanden waren.

Insbesondere betrifft die vorliegende Erfindung
(1) ein Verfahren zur Analyse komplexer Mischungen von Biopolymeren aus einer oder mehreren Proben, das die folgenden Schritte umfasst:
   (a) Spaltung der Biopolymere mittels eines oder mehrerer chemischer Reagenzien und/oder eines oder mehrerer Enzyme in Fragmente;
   (b) Kopplung aller oder eines Teils der in Schritt (a) erhaltenen Biopolymerfragmente und/oder Biopolymere durch kovalente Bindung an einen Linker, der bereits an eine Ankergruppe auf einem unlöslichen Trägermaterial gebunden ist;
   (c) Waschen, wobei die im Schritt (b) nichtgekoppelten Biopolymerfragmente und/oder Biopolymere von den im Schritt (b) an das feste Trägermaterial gekoppelten Biopolymerfragmenten und/oder Biopolymeren durch Waschen mit einem geeigneten Lösungsmittel abgetrennt werden;
   (d) Entkopplung der an das unlösliche Trägermaterial gekoppelten Biopolymerfragmente und/oder Biopolymere durch Spaltung der Bindung zwischen Linker und Biopolymerfragment und/oder Biopolymer oder einer Bindung innerhalb des gekoppelten Biopolymerfragmentes und/oder Biopolymers;
   (e) kovalente Bindung einer Markierung an bestimmte oder an alle in Schritt (c) nicht gekoppelten oder in Schritt (d) entkoppelten Biopolymerfragmente mittels Markierungsreagenzien, wobei die Markierung eine Detektion des Biopolymerfragmentes aufgrund der chemischen oder physikalischen Eigenschaft(en) erlaubt, die das Biopolymer oder eines oder mehrere seiner Fragmente ohne Markierung nicht oder messbar verschieden ausgeprägt aufweisen, und
   (f) Trennung der in Schritt (e) markierten Biopolymerfragmente aufgrund ihrer charakteristischen physikochemischen Eigenschaften und unter Detektion der Markierung, und
(2) einen Kit zur Analyse komplexer Mischungen von Biopolymeren aus einer oder mehreren Proben, umfassend wenigstens einen der folgenden Bestandteile:
   (a) ein oder mehrere chemische Reagenzien oder Enzyme zur Spaltung der Biopolymere in Fragmente wie in (1) definiert;
   (b) ein oder mehrere Reagenzien zur Ausführung der Kopplung, wie in (1) beschrieben;
   (c) ein oder mehrere unlösliche Trägermaterialien gemäß (1);
   (d) einen oder mehrere Linker gemäß (1);
   (e) ein oder mehrere Lösungsmittel zum Waschen gemäß (1);
   (f) ein oder mehrere Reagenzien zur Ausführung der Entkopplung, wie in (1) definiert;
   (g) ein oder mehrere Reagenzien zur kovalenten Bindung einer Markierung, wie in (1) definiert, und
   (h) eine oder mehrere Vorrichtungen zur Trennung, wie in (1) definiert.

### Beschreibung der Figuren

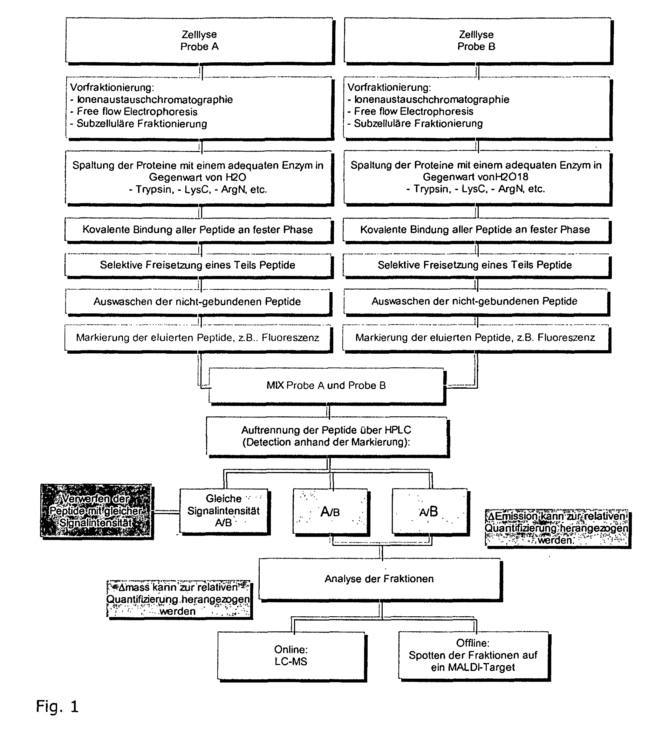

- Fig. 1:: Schematische Darstellung des Verfahrens "selektive Peptid-Exklusions-Chromatographie" zur Proteomanalyse.

### Detaillierte Beschreibung der Erfindung

Das Verfahren der vorliegenden Erfindung soll die Vorteile verschiedener proteomanalytischer Methoden kombinieren und zudem eine (relative) Quantifizierung der Komponenten gestatten. Weiterhin soll die Komplexität des Analytgemisches durch intelligente Auswahl einer Kombination an Kopplungsreagenzien reduziert werden, ohne den Gesamtinformationsgehalt zu verringern. Diese Ziele werden wie folgt erreicht:

Das komplexe Gemisch der Biopolymere wird mit Hilfe enzymatischer oder chemischer Reaktion(en) fragmentiert. Von den resultierenden Fragmenten wird ein spezifischer Teil durch kovalente Bindung mithilfe einer geeigneten chemischen Reaktion an ein unlösliches Trägermaterial gebunden. Dies erlaubt die Abtrennung der gebundenen Fragmente. Im Speziellen werden alle Fragmente, die Bindungsgruppen besitzen (>99.9%), an ein unlösliches Trägermaterial gebunden. Spezifische Fragmente werden durch selektive Entkopplung freigesetzt. Die gebundenen Fragmente werden von der festen Phase gelöst und der weiteren Analyse zugeführt. Es erfolgt eine Markierung (Fluoreszenz, Isotopenmarkierung, chirale oder magnetische Markierung). Die markierten Fragmentgemische mindestens zweier Proben können vereinigt werden. Chromatographische Separation resultiert in der Separation einzelner Fragmente (unabhängig von der Markierung). Diese können nun spektroskopisch (nur quantitative Analyse) und/oder massenspektrometrisch analysiert werden (quantitative Analyse und Fragmentierungsanalyse). Die Eigenschaften der Markierung sind so, dass die Markierung kovalent ist und die Markierungsgruppen bzw. Markierungsverbindungen der (mind.) zwei Proben möglichst identische physikochemische Eigenschaften zeigen, so dass sie die chromatographische Separation in gleichem Ausmaß beeinflussen.

Als "Biopolymer" im Sinne der vorliegenden Erfindung kommen sowohl Proteine und Peptide als auch Nukleinsäurepolymere, Lipide, Zucker und PNAs (peptide nucleic acids = Peptidnukleinsäuren) in Frage. Die Proteine können über alle denkbaren natürlichen Modifikationen verfügen, deren Eigenschaften auch im weiteren Verfahren ausgenutzt werden können.

Als "Komplexe Mischung von Biopolymeren" im Sinne der vorliegenden Erfindung werden Mischungen von Biopolymeren mit mehr als drei Komponenten bezeichnet. "Spaltung" oder "Fragmentierung" der Biopolymere der vorliegenden Erfindung umfasst die Behandlung der Biopolymere mit "Spaltungsreagenzien", so dass "Fragmente" der Biopolymere erhalten werden.

Die "Spaltungsreagenzien" gemäß der vorliegenden Erfindung ermöglichen es, einzelne oder mehrere Aminosäuren und/oder Nukleotide und/oder deren Derivate (z.B. natürliche Modifikationen infolge Glykosylierung) einzeln oder zusammenhängend (als Peptid oder Nukleinsäure) selektiv d.h. an bestimmten Positionen der jeweiligen Sequenz oder unselektiv von dem ursprünglichen Biopolymer abzuspalten. Im Falle der Proteine zählen zu den Spaltungsreagenzien insbesondere Endoproteasen durch deren katalytische Wirkung Proteine in Peptide zerlegt werden. Im Sinne der vorliegenden Erfindung geeignete Endoproteasen sind Trypsin, Submaxillarus-Protease, Chymotrypsin, Staphylococcus-aureus-V8-protease, Asp-N-Protease, Pepsin Lys-C, Glu-C, Arg-C-Proteinase, Asp-N-Endopeptidase, BNPS-Skatole, Caspasen, Chymotrypsin, Clostripain, Factor Xa, Glutamylendopeptidase, GranzymeB, Proline-Endopeptidase, Proteinase K, Staphylococcus-Peptidase I, Thermolysin, Thrombin, Carboxypeptidasen und eine Kombination derselben. Als Reagenzien für eine chemische Spaltung sind beispielsweise CNBr, Ameisensäure, Iodosobenzoesäure, NTCB (2-Nitro-5-thiocyanobenzoesäure), Hydroxylamin, Säurehydrolyse usw. zu nennen, die zentraler Bestandteil bei der Erzeugung von Fragmenten aus Biopolymeren ohne den Einsatz von Endoproteinasen sind. Zur Spaltung von Nukleinsäuren und PNAs können z. B. folgende Endonukleasen zum Einsatz kommen:
Aat II, Acc65 I, Acc I, Aci I, Acl I, Afe I, Afl II, Afl III, Age I, Ahd I, Alu I, Alw I, AlwN I, Apa I, ApaL I, Apo I, Asc I, Ase I, AsiS I, Ava I, Ava II, Avr II, Bae I, BamH I, Ban I, Ban II, Bbs I, Bbv I, BbvC I, BceA I, Bcg I, BciV I, Bcl I, Bfa I, BfrB I, BfuA I, Bgl I, Bgl II, Blp I, Bme1580 I, BmgB I, Bmr I, Bpm I, BsaA I, BsaB I, BsaH I, Bsa I, BsaJ I, BsaW I, BsaX I, BseR I, Bsg I, BsiE I, BsiHKA I, BsiW I, Bsl I, BsmA I, BsmB I, BsmF I, Bsm I, BsoB I, Bsp1286 I, BspCN I, BspD I, BspE I, BspH I, BspM I, BsrB I, BsrD I, BsrF I, BsrG I, Bsr I, BssH II, BssK I, BssS I, BstAP I, BstB I, BstE II, BstF5 I, BstN I, BstU I, BstX I, BstY I, BstZ17 I, Bsu36 I, Btg I, Bts I, Cac8 I, Cla I, Dde I, Dpn I, Dpn II, Dra I, Dra III, Drd I, Eae I, Eag I, Ear I, Eci I, EcoN I, EcoO109 I, EcoR I, EcoR V, Fau I, Fnu4H I, Fok I, Fse I, Fsp I, Hae II, Hae III, Hga I, Hha I, Hinc II, Hind III, Hinf I, HinP1 I, Hpa I, Hpa II, Hpy188 I, Hpy188 III, Hpy99 I, HpyCH4III, HpyCH4IV, HpyCH4V, Hph I, Kas I, Kpn I, Mbo I, Mbo II, Mfe I, Mlu I, Mly I, Mnl I, Msc I, Mse I, Msl I, MspA1 I, Msp I, Mwo I, Nae I, Nar I, Nci I, Nco I, Nde I, NgoM IV, Nhe I, Nla III, Nla IV, Not I, Nru I, Nsi I, Nsp I, Pac I, PaeR7 I, Pci I, PflF I, PflM I, Ple I, Pme I, Pml I, PpuM I, PshA I, Psi I, PspG I, PspOM I, Pst I, Pvu I, Pvu II, Rsa I, Rsr II, Sac I, Sac II, Sal I, Sap I, Sau3A I, Sau96 I, Sbf I, Sca I, ScrF I, SexA I, SfaN I, Sfc I, Sfi I, Sfo I, SgrA I, Sma I, Sml I, SnaB I, Spe I, Sph I, Ssp I, Stu I, Sty I, Swa I, Taq I, Tfi I, Tli I, Tse I, Tsp45 I, Tsp509 I, TspR I, Tth111 I, Xba I, Xcm I, Xho I, Xma I, Xmn I, N.BstNB I, N.Alw I, I-Ceu I, I-Sce I, PI-Psp I, PI-Sce I, McrBC-Endonuclease, McrBC etc.

Im Falle von Zuckern können Amylasen, Maltasen und Lactasen eingesetzt werden.

Unter "Fragmenten" werden im Sinne der vorliegenden Erfindung alle Verbindungen verstanden, die aus der Spaltung eines Biopolymers mit Hilfe von Enzymen oder chemischen Reagenzien entstehen, und die insbesondere zu einer oder mehreren Verbindungsklassen ausgewählt aus der Gruppen der Aminosäuren, Peptide, Nukleotide, Nukleinsäuren, Lipide, Zucker und deren Derivate gehören. Unter Derivaten werden insbesondere Fragmente verstanden, die eine Markierung tragen oder die aus einer Blockierung resultieren. "Blockierung" im Sinne der vorliegenden Erfindung bedeutet, dass bestimmte Monomere an der Position n innerhalb der Sequenz des Biopolymers durch chemische Reagenzien oder Enzyme derivatisiert werden, so dass die Verknüpfung dieses blockierten Monomers zu dem in der Sequenz unmittelbar vorangehenden (an der Position n-1) und/oder nachfolgenden Monomeren (an der Position n+1) bei der Fragmentierung nicht gespalten wird. Der Begriff "Blockierung" kann weiterhin bedeuten, dass vor der Kopplung eine Derivatisierung eines oder beider endständiger Monomeren (Termini) des Biopolymers durch chemische Reagenzien oder Enzyme erfolgt, so dass die blockierten Termini bei der Kopplung nicht an das feste Trägermaterial gekoppelt werden. Als chemische Reagenzien zur Blockierung eignen sich insbesondere Säurehalogenide, Säureanhydride, Aldehyde, Isocyanat-Derivate, Isothiocyanat-Derivat, Succinimid-Derivate, Imidazolyl-Carbamat-Derivate, Trauts-Reagenz-Derivate, Sulfonsäurechlorid-Derivate, Oxiran-Derivate, Imidate, Hydrazide, Sufosuccinimidyl-Derivate, Diimid-Derivate, Maleimid-Derivate, 7-Sulfobenzofurazan-Derivate, insbesondere Acetylchlorid und Citraconsäureanhydrid.

Die "Kopplung" im Sinne der vorliegenden Erfindung ist eine chemische Reaktion, bei der das Biopolymer oder eines oder mehrere seiner Fragmente an ein geeignetes "unlösliches Trägermaterial" kovalent gebunden wird, wobei die Bindung an das unlösliche Trägermaterial bevorzugt über einen Linker erfolgt, der über eine Ankergruppe an das unlösliche Trägermaterial gebunden ist. Bei der kovalenten Kopplung werden vorzugsweise unter reduktiven Bedingungen stabile Verbindungen erzeugt, so z. B. Amide, Ester, Kohlenstoff-Heteroatom-Bindungen etc. (jedoch keine S-S-Bindungen).

Das "unlösliche Trägermaterial" oder die "feste Phase" im Sinne der vorliegenden Erfindung umfassen ein geeignetes, im verwendeten Lösungsmittel unlösliches Material (z. b. aktivierte Glasoberflächen, Magnetic Beads und Polymermaterialien mit funktionalen Gruppen) wie es im Stand der Technik für Festphasensynthesen von Peptiden und Nukleinsäuren bekannt ist, insbesondere ein Harz, wie z. B. Polystyrol. Das unlösliche Trägermaterial ist bevorzugt mit einer geeigneten Ankergruppe versehen, welche eine unter den Bedingungen des erfindungsgemäßen Verfahrens reaktive funktionelle Gruppe ist, so dass ein geeigneter Linker an die Ankergruppe gebunden werden kann.

Als "Ankergruppe" im Sinne der vorliegenden Erfindung eignen sich alle Formen funktioneller Gruppen, die zu einem aktivierten Trägermaterial führen, insbesondere -NH₂, -SH, -Hydrazide, Tosyl, Tresyl, Imidazolylcarbamat, 5-Thiol-2-nitrobenzoesäure-Gruppen oder auch CNBr-aktiviertes Trägermaterial.

Der "Linker" im Sinne der vorliegenden Erfindung ist eine Verbindung mit zwei gleich- oder verschiedenartigen, unter den Bedingungen des erfindungsgemäßen Verfahrens reaktiven funktionellen Gruppen, wovon die eine funktionelle Gruppe X¹ die Bindung an die Ankergruppe und die andere funktionelle Gruppe X² die Bindung an das Biopolymer oder eines oder mehrere seiner Fragmente ermöglicht. Als funktionelle Gruppen für den Linker eignen sich erfindungsgemäß zwei gleich- oder verschiedenartige funktionelle Gruppen X¹ und X². Diese können aus -NH₂, -CN, -OH, -COOH, -COCI, -CON₃, -CHO, -NN, -SH, -SCH₃, -NNH, -CHCH₂, -NCS, -NCO, -CNO, -CNS, -SO₂Hal, -OPO₃²⁻, Oxiran und Vinylsulfon bestehen. Geeignet sind auch gemischte Anhydride und Aktivester, wobei X¹ und/oder X² ausgewählt sind aus -C(O)OR, wobei R aus R¹C(O)-, ortho-Nitrophenyl, -C(NR¹)(NHR¹), N-Oxysuccinimid und 1-Oxybenzotriazol, und R¹ aus Niederalkyl, Cycloalkyl, Aryl, Alkenyl und Alkinyl ausgewählt ist.

Der Linker kann insbesondere die Formel X¹-(A)ₙ-X² aufweisen, wobei A eine Aryl-, Heteroaryl-, Alkyl-, CH₂-Struktur Silyl, Ether, Thioether umfasst, n eine natürliche Zahl von 1 bis 20 ist, und X¹ und X² wie oben definiert sind. Besonders bevorzugt als Linker ist 1,4-Diisothiocyanatobenzol (*para*-Diisothiocyanatobenzol, *p*DITC).

Das gekoppelte Biopolymer im Sinne der vorliegenden Erfindung kann dabei die folgende Formel aufweisen 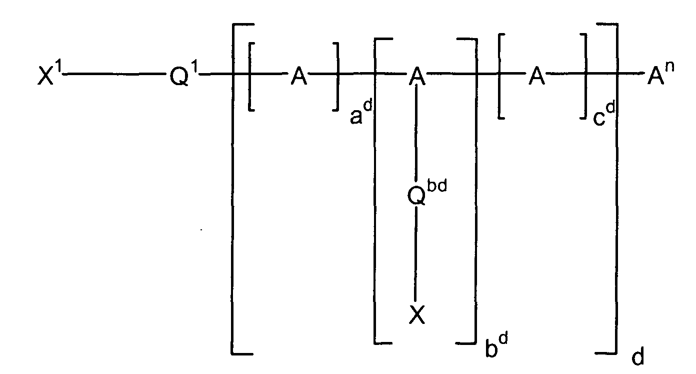 wobei
X = eine feste Phase
A = eine Aminosäure oder Aminosäurederivat oder Monomer eines anderen Biopolymers (Zucker; Lipid, Nukleosid/-tid, etc.)
Q = ein Linker
a ≥ 0
b ≥ 0
c ≥ 0
d ≥ 0
n ≥ Σ_{d}(a^{d}+b^{d}+c^{d})
ist.

Hierbei ist Q vorzugsweise ein mono- oder bifunktioneller Linker, der kovalent an die Aminosäure (oder den jeweiligen Monomeren Baustein des Biopolymers) und/oder kovalent an die feste Phase geknüpft ist. Die Verknüpfung von Q an A kann über eine der folgenden chemischen Bindungen erfolgen: N-N; C-C; N-C; C-S; N-S; S-S; C-O; N-O; S-O; O-O; P-O; P-N; P-C; P-S.

Die Verknüpfung von Q an X kann ebenfalls über eine der nachfolgend genannten chemischen Bindungen erfolgen. Zur Bildung der chemischen Bindung werden die Eigenschaften der jeweiligen Aminosäuren ausgenutzt. Zur Bildung der Verknüpfung verfügt der Linker Q über mindestens eine reaktive Gruppe der Form: -NH₂; -CN; -OH;-COOH; -CHO; -NN; -SH; S-CH₃; -N=NH; -C=CH₂; -N=C=S; -N=C=O; -C=N=O; -C=N=S; -SO₂Cl; -COCI; Oxiran, Vinylsulfon.

Die obengenannte Struktur kann weiterhin mit Markern verknüpft sein ((Fluoreszenz-)Farbstoffe, Isotopenmarkierung, u.ä.). Der Linker kann ein Oligonukleotid, ein PNA oder ein Peptid beinhalten oder sein.

Das "Waschen" umfasst das ein- oder mehrmalige Zugeben und Entfernen von Flüssigkeit oder das kontinuierliche Zugeben und Entfernen von Flüssigkeit, um Bestandteile der Ausgangslösung zu entfernen. Ggf. können diese Flüssigkeiten einer weiteren Analyse zugeführt werden.

"Entkopplung" im Sinne der vorliegenden Erfindung bedeutet, dass die an das unlösliche Trägermaterial gekoppelten Biopolymerfragmente und/oder Biopolymere durch Spaltung der Bindung zwischen A) Trägermaterial und Linker, B) innerhalb des Linkers, C) zwischen Linker und Biopolymer(fragment) und/oder D) innerhalb des Biopolymer(fragment)s vom Trägermaterial abgelöst werden. Unter "Spaltung" versteht man die Spaltung einer oder mehrer der Bindungen 1,2,3 und/oder 4 (Schema 1, A) sowie die Spaltung einer Bindung innerhalb des Linkers (B) und/oder innerhalb des Bioploymers oder Bioploymerfragemnts (C). Dieses wird in dem nachfolgenden Schema 1 näher erläutert. 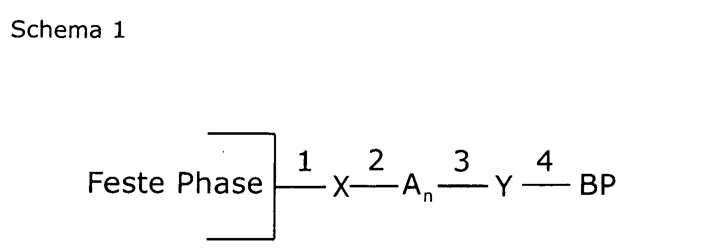
A: Nummerierung der Bindungen bei Kopplung des Biopolymers oder Biopolymerfragmentes über einen Linker im Sinne der Erfindung. 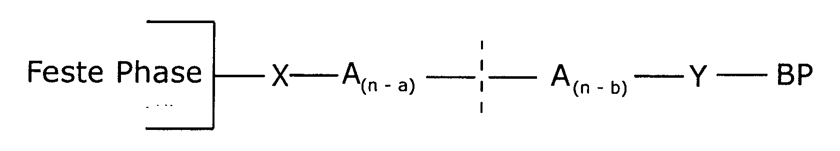
B: Trennung einer Bindung innerhalb des Linkers. A sei eine beliebige der unter PUNKT genannten Molekülgruppen, n eine ganze Zahl, n,a,b<20 und sowie a+b=m. 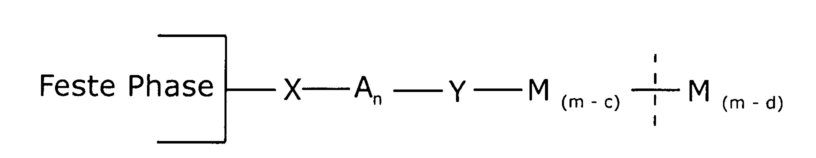
C: Trennung einer Bindung innerhalb des Biopolymers oder Biopolymer-Fragmentes. M seien beliebige unter PUNKT aufgeführte Monomere des Biopolymers oder Biopolymerfragmentes; m,c,d seien natürliche Zahlen ≥0 und c+d=m.

Die "Markierung" erfolgt durch die chemische Reaktion eines Biopolymers oder eines oder mehrerer seiner Fragmente mit dem Markierungsreagenz, wobei das Biopolymer oder eines oder mehrerer seiner Fragmente mit dem Markierungsreagenz gekoppelt wird. Die Markierung versieht das Biopolymer oder eines oder mehrerer seiner Fragmente mit einer chemischen oder physikalischen Eigenschaft, die das Biopolymer oder eines oder mehrerer seiner Fragmente vorher nicht oder nicht so stark d.h. messbar verschieden ausgeprägt aufwies(en) wie mit der Markierung.

Als "Markierungsreagenz" seien beispielhaft (Fluoreszenz-)Farbstoffe genannt, aber auch insbesondere isotopenmarkierte, chirale oder magnetische Verbindungen, wobei auch Kombinationen der genannten Markierungseigenschaften in demselben Markierungsreagenz erfindungsgemäß möglich sind.

Unter "Trennung" wird die Trennung der einzelnen markierten Fragmente mittels elektrophoretischer und/oder chromatographischer Methoden, bevorzugt mittels Flüssigkeitschromatographie (LC), besonders bevorzugt mittels Hochleistungsflüssigkeitschromatographie (HPLC) an Umkehrphasen wie C18-Reverse-Phase oder Ionenaustauschchromatographie durchgeführt.

"Charakterisierung" und "Identifizierung" umfasst die Strukturaufklärung der markierten Fragmente mit spektroskopischen Methoden, wozu die Massenspektroskopie, NMR-, UV-, VIS-, IR-Spektroskopie geeignet sind.

"Vorrichtung zur Trennung" bedeutet ein System zur Flüssigkeitschromatographie, Kapillareletrophorese, Zonenelektrophorese, Gelelektrophorese, Freeflow-Elektrophorese, Extraktion.

"Vorrichtung zur Charakterisierung" bedeutet ein oder mehrere Geräte zur Detektion der eingeführten Markierung (z. B. Fluoreszenzdetektor, Absorptionsspetrometer, Multiphotondetektor) und/oder der Struktur eines Biopolymers oder Biopolymerfragments (Massenspektrometer, NMR).

Die Ausführungsform A des Verfahrens der vorliegenden Erfindung setzt die "selektive Peptid-Exklusions-Chromatographie" zur Proteomanalyse ein und umfasst die folgenden Schritte (siehe auch die schematische Darstellung in Fig. 1):
A.1. Fraktionierung der komplexen Biopolymergemische:
   Dieser Schritt erfolgt optional, je nach Komplexität des Analyten bzw. Trennkapazität der nachfolgenden Schritte. Als Methoden kommen chromatographische Verfahren ebenso in Frage wie physikochemische Trennmethoden.
A.2. Spaltung der Biopolymere:
   Die Biopolymere der komplexen Mischung werden fragmentiert. Dies geschieht z.B. durch den Einsatz von Enzymen. Eine weitere Möglichkeit besteht in der chemischen Fragmentierung der zu analysierenden Biopolymere, z.B. mit CNBr oder partieller saurer Hydrolyse.
   Die Spaltung des Biopolymers oder Gemisches von Biopolymeren kann zum einen mit Hilfe der katalytischen Wirkung anderer gereinigter Biopolymere geschehen oder durch Aktivierung in der Lösung vorhandener Biopolymere.
   Werden gereinigte Biopolymere verwendet, so können diese sowohl in löslicher Form als auch an ein unlösliches Material gekoppelt eingesetzt werden.
   Eine weitere Möglichkeit ist der Einsatz chemischer Reagenzien zur Spaltung der in der Mischung enthaltenen Biopolymere. Hierbei sei beispielhaft der
   Einsatz von CNBr genannt, durch das Proteine selektiv aufgrund ihrer Primärsequenz in Fragmente gespalten werden.
A.3. Retardierung der Biopolymerfragmente:
   Aus Schritt 2 resultierende Fragmente werden kovalent an ein unlösliches Trägermaterial gekoppelt. Hierbei kann die Natur der Kopplungsreaktion spezifisch oder unspezifisch sein, d.h. es können alle oder nur ein Teil der aus Schritt 2 resultierenden Fragmente an das Material gekoppelt werden. Als Kopplungsreagenz kommen homo- oder heterobifunktionelle Reagenzien zum Einsatz. Durch die Kopplung an das unlösliche Trägermaterial ist es möglich, die Biopolymerfragmente in unlöslicher Form zu handhaben.
   Die Kopplung wird mit Hilfe chemischer Reagenzien und geeigneter Reaktionsbedingungen erreicht. Hierbei kann die Art der Kopplung auf eine spezielle Eigenschaft des Biopolymers abgestimmt sein oder selektiv über einen bestimmten Baustein des Biopolymers erfolgen. Die Kopplung kann mit einem mono- oder hetereobifunktionellen Reagenz durchgeführt werden. Hierbei sei es gleichgültig, ob die Kopplung des Linkers erst an ein unlösliches Trägermaterial und dann an ein Biopolymer erfolgt, oder in umgekehrter Reihenfolge durchgeführt wird. Als Kopplungsreagenz sei beispielhaft Phenylendiisothiocyanant genannt.
   Das Kopplungsreagenz kann zudem direkt eine Gruppe enthalten, die das Biopolymer in eindeutiger Weise markiert, sei es aufgrund seiner spezifischen physikochemischen Eigenschaften oder seiner Masse oder anderer Charakteristika. Die folgenden Verfahren können in Schritt A.3 zum Einsatz kommen:
   a) Beispiele zur Umsetzung von Aminen (-NH₂)
      - Umsetzung mit Anhydriden: 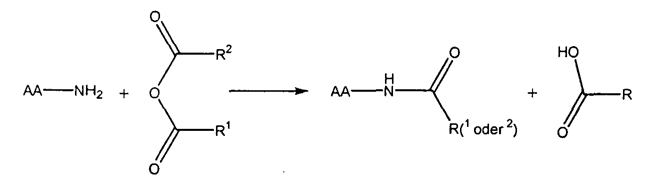
      - Succinimidester 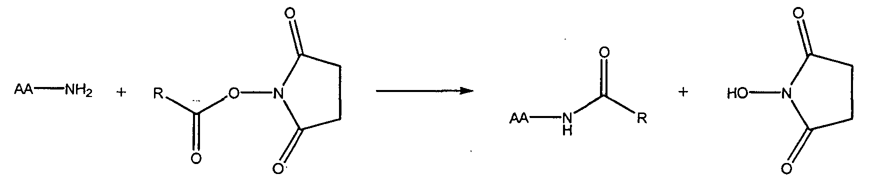
      - Aminidierung 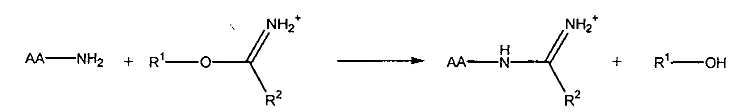
      - (Reduktive) Alkylierung 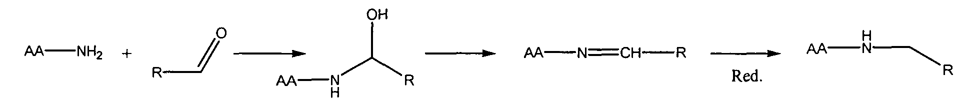
      - Halogen-Carbamate, z.B. B. 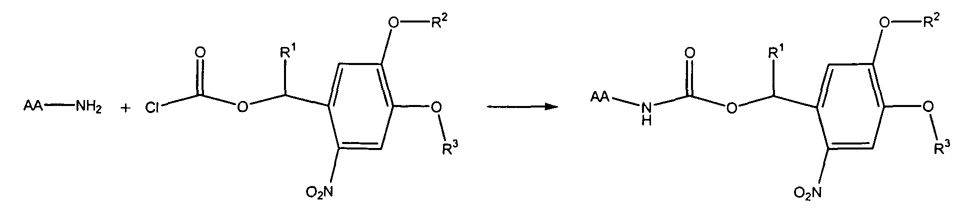
   b) Beispiele zur Umsetzung von Thiolen
      - Dithiole wie 5,5'-Dithiobis(2-nitrobenzoesäure) (DTNB, Ellman-Reagenz) 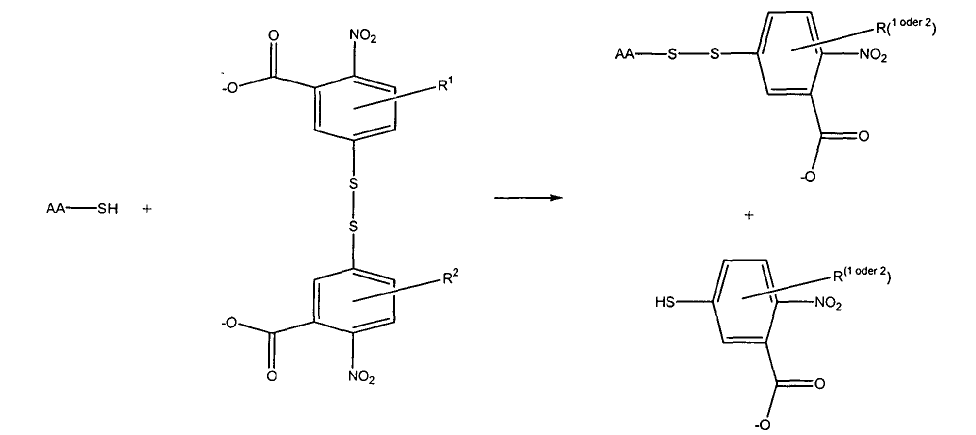
      - Umsetzung mit Halogen-Quecksilber-Verbindungen 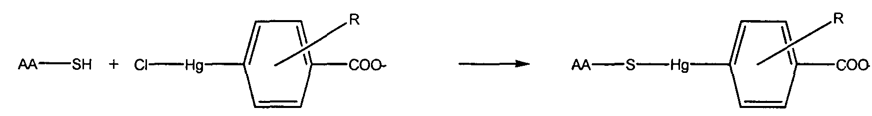
      - Umsetzung mit 2-Nitro(hydroxy)benzylbromid 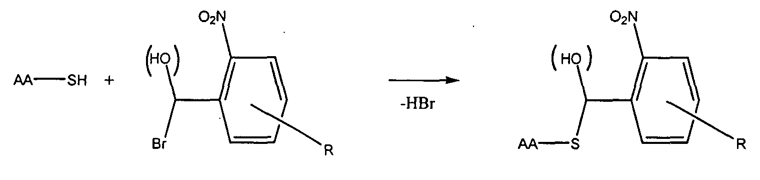
   c) Beispiele zur Umsetzung von Glutamat-Resten oder Aspartat-Resten (CarboxyGruppen)
      - Umsetzung mit Carbodiimiden 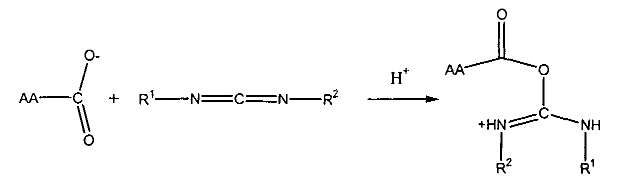
   d) Beispiele für die Umsetzung von Argininresten
      - Umsetzung mit Glyoxal (Derivaten) 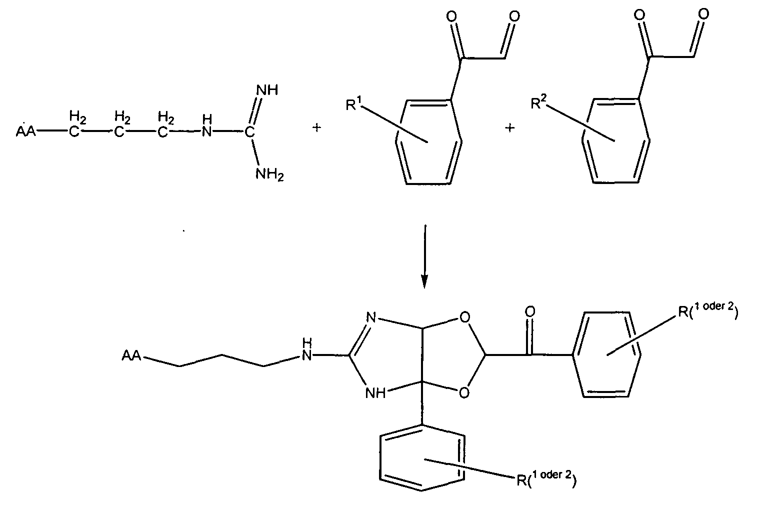
   e) Beispiele für die Umsetzung von Tyrosindresten
      - Koshlands-Reagenz (Derivate) 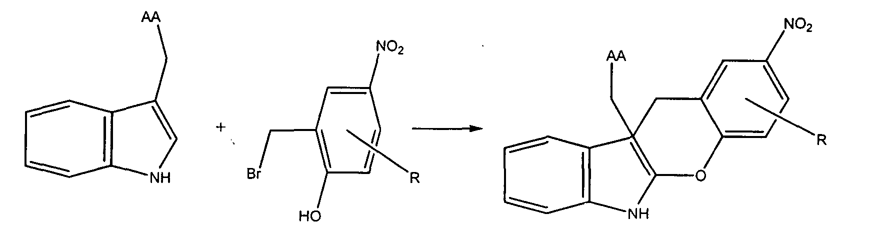
      - Umsetzung mit Sulfenylhalogenide 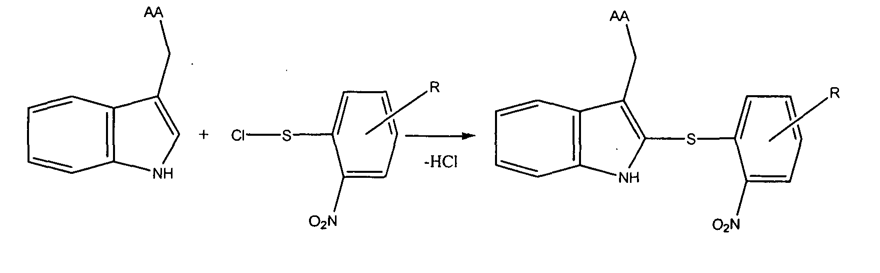
   f) Beispiele für die Umsetzung von Methionin mit Iodacetamid-Derivaten (reversibel) 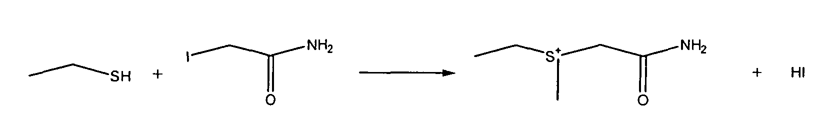
   g) Beispiele für die Umsetzung von Histidinresten
      - Umsetzung mit Diethylpyrocarbamat (Derivaten) 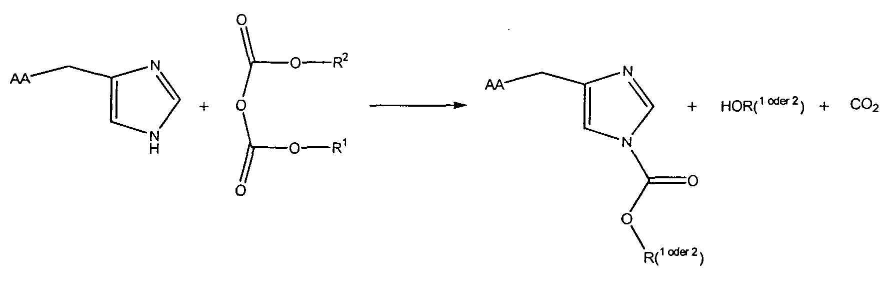 Ebenfalls Kopplungsreagenzien im Sinne der Erfindung sind Silicium-Verbindungen des folgenden Typs, sofern sie zur Funktionalisierung von Glasoberflächen (Matrix) eingesetzt werden: 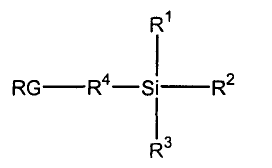
         a. Hierbei sind in erster Linie Verbindungen gemeint, bei denen R¹=R²=R³= -O-(CH₂)ₙH, mit n≥1 und R⁴ = -(CH₂)ₘH mit m≥1. Weiterhin sei RG eine reaktive im sinne von funktionelle Gruppe wie oben beschrieben, mit deren Hilfe entweder ein Linker oder ein Biopolymer oder Biopolymerfragment an die Silan-Verbindung gebunden werden kann.
         b. Hierbei sei R jeweils ein beliebiger Rest, der entweder weitere funktionelle Gruppen trägt und/oder an eine Matrix im Sinne der Erfindung gekoppelt ist.
         c. Sind in den Beispielen mehrere Reste vorhanden (R², R³, etc.), so seien diese beliebig unterschiedlich oder identisch. Weiterhin entspreche jeder der Reste den unter a) genannten Bedingungen.
A.4. Entkopplung der Biopolymere:
   Die an das unlösliche Trägermaterial gekoppelten Biopolymere können selektiv oder unspezifisch entkoppelt werden. Hierbei kommt sowohl die Natur der Kopplung als auch die bei der Erzeugung der Fragmente eingesetzte Methode zum Tragen. Durch Auswahl der Fragmentierungs-, Kopplungs- und Entkopplungsmethode wird die Komplexität des Gemisches an Biopolymeren reduziert. Eine Entkopplung von Biopolymeren kann wie im Schema 1 dargestellt zwischen Trägermaterial und Linker, innerhalb des Linkers zwischen Linker und Bioploymer(fragment) und innerhalb des Biopolymer(fragment)s erfolgen. Letztes ist durch Endoprotease-Spaltung oder CNBr-Spaltung möglich.
A.5. Markierung der Fragmente:
   Die Fragmente der Biopolymere werden mit Markierungen versehen, die eine anschließende Zuordnung zu der Ausgangsprobe erlauben.
A.6. Analyse der markierten Biopolymerfragmente:
   Die markierten Biopolymerfragmente werden anhand charakteristischer physikochemischer Eigenschaften voneinander getrennt. Hierbei kann zuvor eine Vermischung markierter Biopolymerfragmente aus verschiedenen Proben erfolgen.
A.7. Analyse der getrennten Biopolymerfragmente:
   Hierzu kann schon die Trennung der Biopolymerfragmente (Schritt 6) gezählt werden. Weitere Analysemethode ist in erster Linie die Massenspektrometrie, mit Hilfe derer eine exakte Charakterisierung der Biopolymerfragmente erfolgt. Diese erlaubt eine Zuordnung zum ursprünglichen Biopolymer.

Die Schritte A.2, A.3, A.5 können je nach Bedarf in unterschiedlicher Reihenfolge durchgeführt werden. So ist z.B. eine Markierung vor der Fragmentierung denkbar, durch die eine funktionelle Gruppe zur kovalenten Bindung des Biopolymers eingeführt wird. Die kovalente Kopplung wiederum kann sowohl vor wie auch nach der Fragmentierung durchgeführt werden.

Auch in Schritt a.3 nicht gebundene Biomoleküle können einer weiteren Markierung und Analyse ab Schritt A.5 zugeführt werden.

Neben der Markierung von Biopolymeren in Schritt A.5 ist zusätzlich auch eine Markierung der Biopolymere während der Spaltung (A.2) durch Einsatz von schwerem Wasser und/oder O 18 -Wasser möglich. Diese Variante kann bei einer Differentialanalyse zweier Ausgangsproben ggf. anstelle einer Markierung durch A.5 eingesetzt werden, indem eine Proben in Anwesenheit von H2O und die andere in Anwesenheit von schwerem Wasser oder O 18 -Wasser oder schwerem O 18 -Wasser gespalten wird. Die Analyse erfolgt dann in den Schritten A.6 und A.7 durch Massenspektrometrie.

In der bevorzugten Ausführungsform B betrifft die vorliegende Erfindung ein Verfahren zur Isolierung von Peptiden aus komplexen Protein-, Peptid- oder anderen Gemischen, das die nachfolgend gezeigten Schritte B.1 bis B.6 mit der Reaktionsfolge Spaltung-Kopplung-Entkopplung der Biopolymergemische umfasst. Diese Ausführungsform ist zur Analyse von nicht lysinhaltigen Peptiden aus fragmentierten Proteinen geeignet. Sie hat den Vorteil, dass im ersten Schritt (B.4) blockierte N-terminale Fragmente von Proteinen isoliert und einer weiteren differentiellen Analytik zugeführt werden können. In Schritt B.5 werden alle lysinfreien Peptide entkoppelt und einer weiteren differentiellen Analytik zugeführt. Dies hat den Vorteil, dass bei einer deutlich höheren Proteinabdeckung als bei dem oben beschriebenen Verfahren ICAT nur eine reduzierte Anzahl an Peptiden pro Protein der differentiellen Analytik zugeführt werden müssen.
B.1. Spaltung der Proteine im Proteingemisch
B.2. Aktivierung der festen Phase, die mit einer -NH₂-Funktionalität versehen ist, mit *p*DITC: 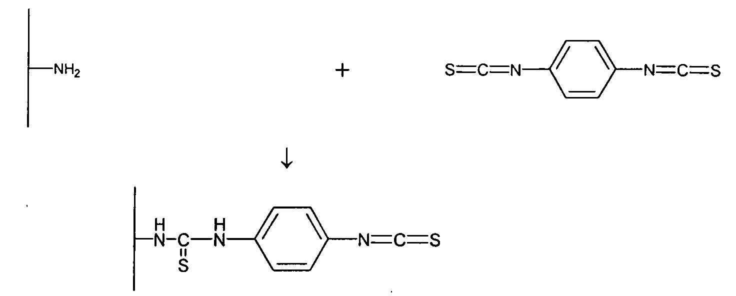
B.3. Kopplung der Peptide an das unlösliche Trägermaterial: 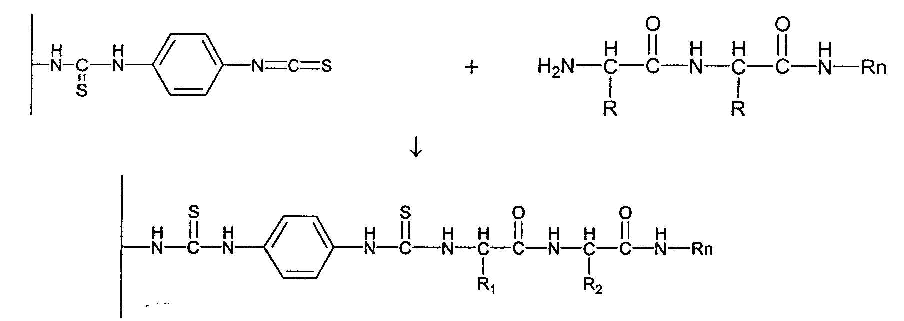
B.4. Waschschritte (Auffangen der nichtgekoppelten Peptide)
B.5. Entkopplung: 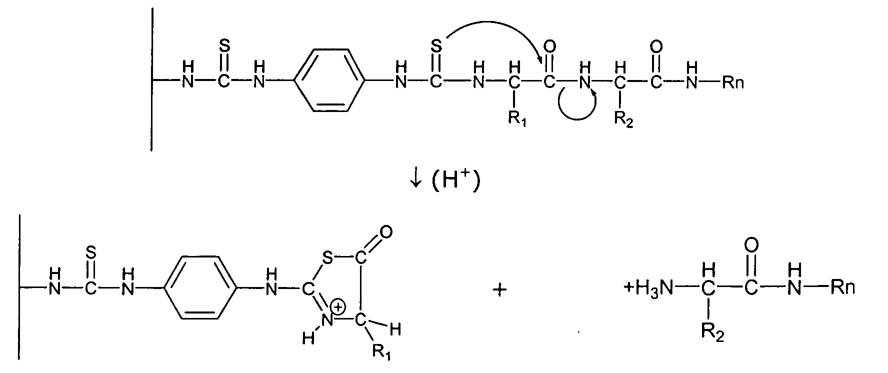
B.6. Markierung:
   Im weiteren Verlauf des Verfahrens werden die entkoppelten und eluierten Peptide markiert. Dies geschieht vorzugsweise über die zwangsläufig vorhandenen freien N-Termini mit einem Markierungsreagenz.

In Schritt B.3 werden alle Peptide mit einem freien N-Terminus an die Matrix gekoppelt. Die in Schritt B.4 anfallenden nichtgekoppelten Peptide entsprechen also den blockierten N-Termini derjenigen Proteine, welche in der Sequenz ein Arginin vor einem Lysin tragen. Diese werden ebenfalls isoliert und analysiert, da sie einen Großteil der anfänglich eingesetzten Proteine repräsentieren.

In Schritt B.4 werden zudem die nicht-gekoppelten Biopolymerfragmente isoliert und der weiteren Analyse zugeführt.

In Schritt B.5 können nur diejenigen Peptide entkoppelt werden, die ausschließlich über einen freien N-Terminus angekoppelt sind d.h. die Entkopplung im Schritt B.5 erfolgt unter diesen Bedingungen nur, wenn die Bildung eines ATZ (Anilinothiazolidinon) möglich ist, sodass lediglich diejenigen Peptide freigesetzt werden, welche ausschließlich über einen freien N-Terminus an das unlösliche Trägermaterial gekoppelt sind. Peptide, welche über Aminosäureseitenketten mit NH₂-Funktionalität (Lys) an das unlösliche Trägermaterial gekoppelt sind, können mittels dieser Reaktionsfolge nicht freigesetzt werden. Die geschickte Kombination von Spaltungsreagenz und Kopplungsreagenz ermöglicht also eine selektive Reduktion des Peptidgemisches auf eine überschaubare Menge an Peptiden.

Als Markierungsreagenz für den Schritt B.6 ist im folgenden beispielhaft eine Fluoreszenzmarkierung gezeigt. Die physikochemischen Eigenschaften der Fluoreszenzmarker sollten sich nicht gravierend unterscheiden wohingegen ihre Emissionsmaxima möglichst weit auseinander liegen sollten.

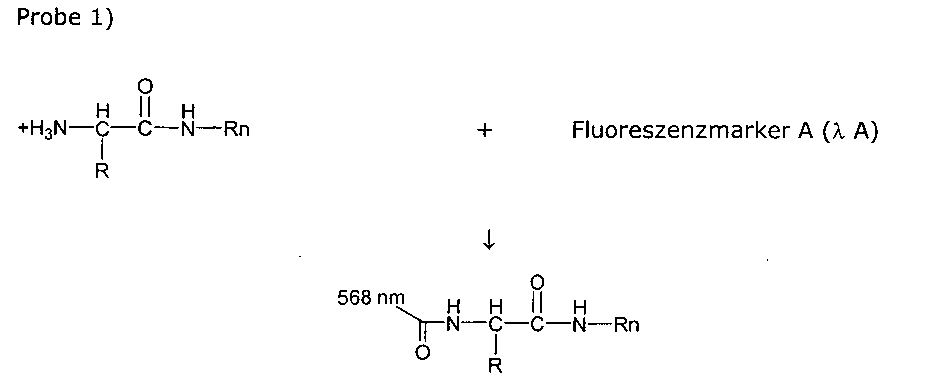

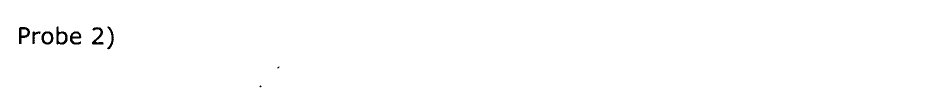

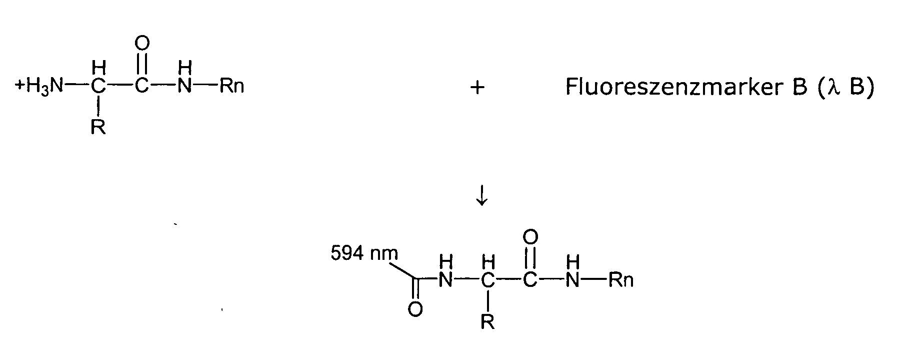

Die so markierten Proben werden nun vereinigt und via LC getrennt, so dass idealerweise pro Fluoreszenzpeak identische Peptide koeluieren.

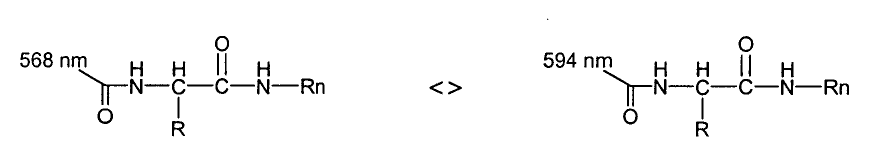

Dank der Fluoreszenzmarkierung kann nun das relative Verhältnis der Peptide zueinander bestimmt werden und nur diejenigen Fraktionen der Analyse zugeführt, welche über unterschiedliche Fluoreszenzintensität verfügen. Hierdurch wird die Anzahl der zu analysierenden Fraktionen erheblich reduziert.

Der Umstand, dass blockierte N-Termini mit Argininresten am C-Terminus nicht gekoppelt werden, kann zur weiteren Spezifizierung der Analyse ausgenutzt werden. Die Mischung der Proteine wird beispielsweise synthetisch blockiert, was z.B. durch Acetylierung oder durch Umsetzung mit Citraconsäure oder Citraconsäureanhydrid (wie in Fig. 1 dargestellt) geschehen kann, und anschließend tryptisch (d.h. mit Trypsin) verdaut oder auf andere Weise enzymatisch oder chemisch gespalten. Hierdurch verfügen nur aus der Spaltung resultierende Peptide über einen freien N-Terminus über den sie an eine Matrix gekoppelt werden können. Die blockierten N-terminalen Peptide werden nicht an die feste Matrix gekoppelt und können analysiert werden. Die Proteinabdeckung bei diesem Verfahren sollte theoretisch bei 100 % liegen.

In den weiteren besonders bevorzugten Ausführungsform C des Verfahrens erfolgt die Isolierung der N-Termini aller Proteine einer Probe gemäß der nachfolgend gezeigten Schritte C.1 bis C.4. Charakteristisch für diese Ausführungsform C ist die Analyse von (synthetisch) blockierten N-Termini von Proteinen und die Blockierung z.B. mit Citraconsäureanhydrid. Diese Verfahrensvariante hat den Vorteil, dass nur die N-Termini von Proteinen analysiert werden (d. h. es wird nur ein Peptid pro Protein analysiert bei ca. 100 % Proteinabdeckung, während bei dem obenbeschriebenen ICAT-Verfahren nur alle cysteinhaltigen Peptide analysiert werden). Es ist anzumerken, dass ca. 16 % der Proteine, die in der NCBI-Proteindatenbank verzeichnet sind, kein Cystein enthalten und somit über Cystein-selektive Verfahren nicht nachgewiesen werden können. Die anderen 84 % der Proteine in der NCBI-Proteindatenbank enthalten mindestens ein Cystein und viele enthalten mehrere Cysteine. Dies bedeutet, dass in dem ICAT-Verfahren eine deutlich größere Anzahl an Peptiden analysiert werden müssen, was eine deutlich höhere Komplexität des Verfahrens zur Folge hat.
C.1 Blockierung der N-Termini und Lysinreste durch Umsetzung mit Aldehyden, Isocyanat, Isothiocyanaten, N-hydroxy-Succinimidester-Gruppe, Sulfonsäurehalogenide, aktivierte Ester oder Säureanhydriden, insbesondere Citraconsäureanhydrid z.B. gemäß: 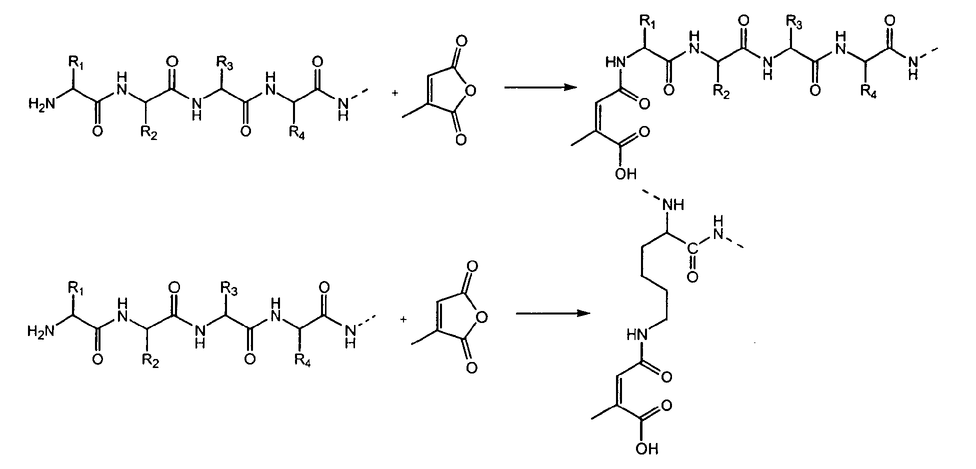
C.2 Spaltung mit Trypsin (nur noch an der C-terminalen Seite von Arg)
C.3 Kopplung an unlösliches Trägermaterial/Matrix (-R-N=C=S)

Hierbei werden also zuerst alle N-Termini blockiert. Nach der Spaltung liegen alle internen und C-terminalen Fragmente wieder mit freiem N-Terminus vor, der an die Matrix gekoppelt werden kann. Die einzigen Peptide, die nicht an die Matrix gekoppelt werden, sind die N-Termini. Diese werden mittels HPLC fraktioniert und im MS analysiert. Eine vorherige Markierung und Mischen verschiedener Proben ist optional.

Es ist aber auch eine weitere Reduktion des Peptidaufkommens möglich, wobei insbesondere die Reaktivität von Methionin ausgenutzt werden kann. So ist es denkbar, die Spaltung der Biopolymere mit anderen Enzymen oder chemisch durchzuführen (kombinierte Spaltung). Hierdurch sollte sich die Anzahl der anfallenden Peptide drastisch ändern. Es ist aber auch denkbar, die analog dem beschriebenen Verfahren an die Matrix gekoppelten Peptide chemisch mit CNBr umzusetzen, so dass in diesem Fall Peptide von der Matrix eluiert werden, die durch Spaltung an Methionin entstehen. Hierdurch kann z.B. eine quasi zweistufige Extraktion der matrixgekoppelten Peptide erfolgen, da nach CNBr-Spaltung nach wie vor die nichtlysinhaltigen Peptide eluiert werden können (durch Abbau analog der Edman-Chemie). Auf diese Weise lässt sich das Verfahren in verschieden sensitive Teilverfahren splitten, die auf verschiedene Organismen oder Fragestellungen angewandt werden können.

Eine weitere Ausführungsform des erfindungsgemäßen Verfahrens besteht in der Ausnutzung der während der CNBr-Umsetzung auftretenden Lacton-Zwischenstufe. Diese kann ebenfalls zur Kopplung der Peptide an eine feste Matrix ausgenutzt werden. Das Homoserinlacton entsteht dabei in folgenden Reaktionsschritten:

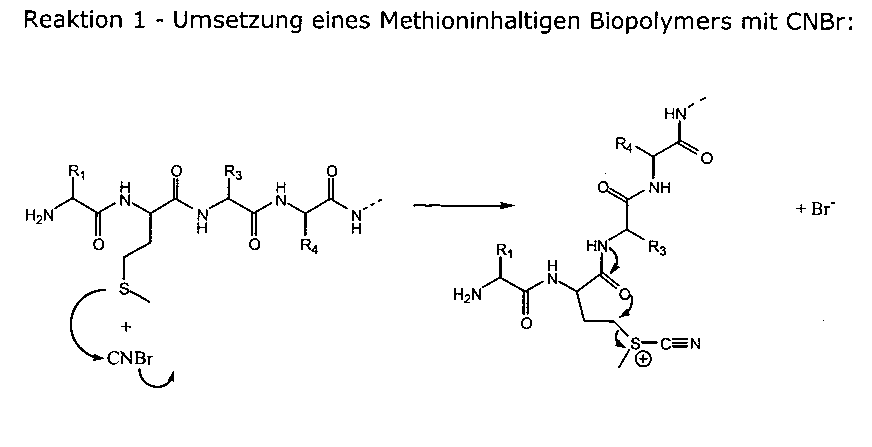

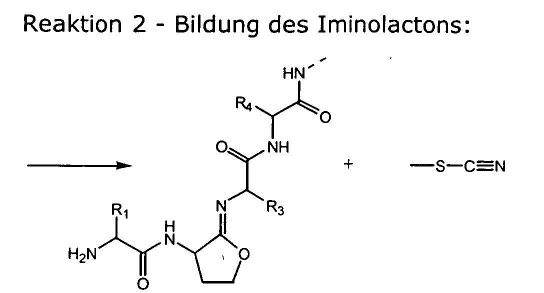

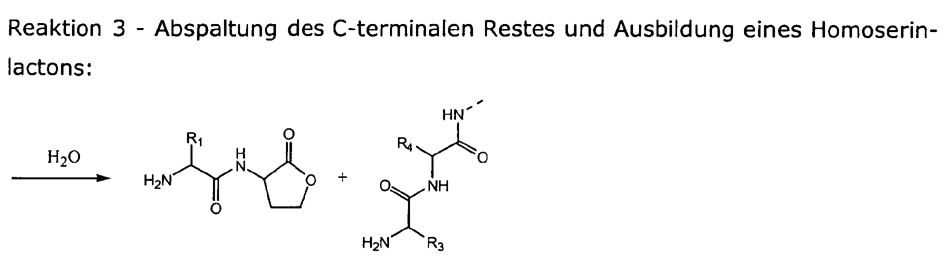

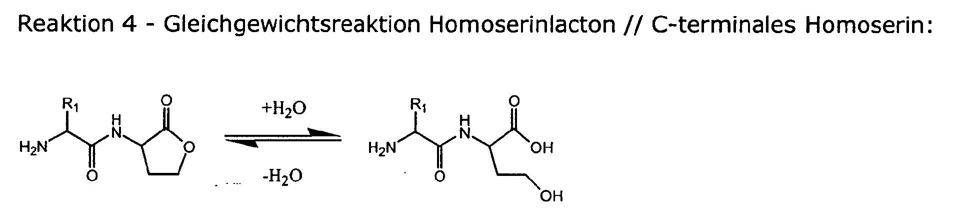

Der (Homoserin-)Lactonring stellt eine aktive Komponente dar und kann mit Aminen umgesetzt werden; eine quantitative Umsetzung wird deshalb sichergestellt, weil hierdurch das Lacton aus dem Gleichgewicht abgezogen wird.

Das erfindungsgemäße Verfahren weist gegenüber dem ICAT-Verfahren folgende Unterschiede auf:
A) Der elementare Unterschied des erfindungsgemäßen Verfahrens gegenüber dem ICAT-Verfahren besteht darin, dass die Biopolymere NICHT mit einem detektierbaren Affinitäts-Tag versehen werden. Die Biopolymere werden aufgrund inhärenter Eigenschaften selektioniert.
B) Reihenfolge der Arbeitsschritte:
   ICAT: Markierung - Mischen - Selektion - Analyse
   Erfindungsgemäßes Verfahren: Selektion - Markierung - Mischen - Analyse
C) Das erfindungsgemäße Verfahren beruht auf einer negativ-Selektion wohingegen ICAT auf einer positiv-Selektion beruht (markierte Peptide werden durch Affinitätschromatographie abgetrennt und dann per LC-MS analysiert).
D) Das erfindungsgemäße Verfahren verfügt über höhere Variabilität,
   die selektive Entkopplung kann chem. oder enzymatisch erfolgen.
   die Kopplung kann ebenfalls selektiv erfolgen.
E) Die Selektion des erfindungsgemäßen Verfahrens erfolgt NICHT über Affinität (Biot / Avidin) sondern durch
   Ausbildung einer kovalenten Bindung (nicht Gleichgewichtsabhängig).
F) Das erfindungsgemäße Verfahren setzt in der Peptidchemie langjährig etablierte und validierte chemische Verfahren ein.

Aufgrund dieser Unterschiede verfügt das erfindungsgemäße Verfahren der vorliegenden Erfindung über zahlreiche Präzisierungsmöglichkeiten bei folgenden Arbeitsschritten:
A) Vorbehandlung der Probe zur Erhöhung der Selektivität (chem. Blockierung aller N-Termini s. bevorzugte Ausführungsform C, chem. Modifikation von Spaltstellen)
B) Generierung der Fragmente mittels enzymatischer oder chemischer Spaltung
C) kovalente Kopplung der Fragmente über andere Funktionalitäten (s. Beispiele)
D) selektive Entkopplung der Fragmente (siehe Schema 1 Matrix-X-Linker- Y-Biopolymer).

Die vorliegende Erfindung wird anhand der folgenden Beispiele näher erläutert, die jedoch nicht so auszulegen sind, dass sie den Umfang der Erfindung einschränken.

### Beispiel:

Reagenzien: NH₂-funktionalisiertes Trägermaterial (z.B. Aminopropyl-Glas, APG) Toluol, pDITC, DMF, THF, TFA, MeOH, Schutzgas, Essigsäure, HCI, Trietylamin (TEA)
Puffer B: 0.2 M Na₂HPO₄, pH 9.0, 1% SDS
Kopplung des Linkers an die Matrix:
   - unter Schutzgas etwa 200 mg DITC in 5 ml trockenem DMF aufnehmen.
   - 2 g Aminopropylglas-Beads zugeben
   - 2 h inkubieren
   - Lösungsmittel absaugen und
   - Glasbeads mit 100 ml Benzol waschen
   - Glasbeads anschließend mit 150 ml wasserfreiem MeOH waschen,
   - im Vakuum trocknen und
   - bei 4°C lagern
Kopplung des Biopolymers an den Linker/Waschen:
   - Biopolymer trocknen
   - Biopolymer in Puffer B resuspendieren
   - geignete Menge APG/DITC-Trägermaterial und TEA zugeben
   - 45 Minuten auf 55°C erhitzen
   - Mit Puffer B, 20% TFA und Wasser ungebundene Bestandteile auswaschen (ggf. zur Analyse mit Trennung und MS).
Entkopplung (analog gängiger Edman-Chemie) und Markierung:
   - Feste Phase (APG-DITC-BP) trocknen
   - Entkopplung mit TFA
   - Flüssige Phase (enthält freie BP bzw. BP-Fragmente) in SpeedVac trocknen
   - umsetzen mit F-SCN (F = fluoreszierender Rest) in THF, 30 min. 52 °C, wobei jede Probe einen anderen flureszierenden Rest erhält (z.B. unterschiedliche) Emissionsmaxima, unterschiedliche Masse, unterschiedliche Isotopenmarkierung)
   - Trocknen
   - Aufnehmen in wässriger Lösung.

Zu vergleichende Proben mischen und der folgenden Trennung und Analyse zuführen.
Trennung: Im folgenden werden die vereinigten Mischungen der markierten Biopolymere oder Biopolymerfragmente mittels chromatographischer oder elektrophoretischer Verfahren getrennt. Die Detektion erfolgt mit geeigneten Detektoren (z.B. Fluoreszenzdetektor und Massendetektor).

## Patentansprüche

1. Verfahren zur Analyse komplexer Mischungen von Biopolymeren aus einer oder mehreren Proben, das die folgenden Schritte umfasst:
(a) Spaltung der Biopolymere mittels eines oder mehrerer chemischer Reagenzien und/oder eines oder mehrerer Enzyme in Fragmente;
(b) Kopplung aller oder eines Teils der in Schritt (a) erhaltenen Biopolymerfragmente und/oder Biopolymere durch kovalente Bindung an einen Linker, der bereits an eine Ankergruppe auf einem unlöslichen Trägermaterial gebunden ist;
(c) Waschen, wobei die im Schritt (b) nichtgekoppelten Biopolymerfragmente und/oder Biopolymere von den im Schritt (b) an das unlösliche Trägermaterial gekoppelten Biopolymerfragmenten und/oder Biopolymeren durch Waschen mit einem geeigneten Lösungsmittel abgetrennt werden und gegebenenfalls einer unabhängigen Analyse zugeführt werden können;
(d) Entkopplung der an das unlösliche Trägermaterial gekoppelten Biopolymerfragmente und/oder Biopolymere durch Spaltung der Bindung zwischen Linker und Biopolymerfragment und/oder Biopolymer oder einer Bindung innerhalb des gekoppelten Biopolymerfragmentes und/oder Biopolymers;
(e) kovalente Bindung einer Markierung an bestimmte oder an alle in Schritt (d) nicht gekoppelten oder in Schritt (d) entkoppelten Biopolymerfragmente mittels Markierungsreagenzien, wobei die Markierung eine Detektion des Biopolymerfragmentes aufgrund der chemischen oder physikalischen Eigenschaft(en) erlaubt, die das Biopolymer oder eines oder mehrere seiner Fragmente ohne Markierung nicht oder messbar verschieden ausgeprägt aufweisen, und
(f) Trennung der in Schritt (e) markierten Biopolymerfragmente aufgrund ihrer charakteristischen physikochemischen Eigenschaften und unter Detektion der Markierung.

2. Verfahren nach Anspruch 1, wobei
(i) die Biopolymere ausgewählt sind aus einem oder mehreren aus der Gruppe der Peptide, Nukleinsäuren, Peptid-Nuklein-Säuren (PNAs), Lipide, Zucker und deren Derivate und/oder die Fragmente ausgewählt sind aus einem oder mehreren aus der Gruppe der Aminosäuren, Peptide, Nukleotide, Nukleinsäuren, Lipide, Zucker und deren Derivate; und/oder
(ii) die kovalente Bindung des Biopolymers an den Linker eine unter reduktiven Reaktionsbedingungen stabile kovalente Bindung ist.

3. Verfahren nach Anspruch 1 oder 2, wobei
(i) vor der Spaltung (Schritt (a)) ein Schritt zur Fraktionierung der komplexen Biopolymergemische nach der Zelllyse durch chemische und/oder physikochemische Trennmethoden, bevorzugt durch ein oder mehrere Verfahren ausgewählt aus subzelluläre Fraktionierung, Fällung, freie Elektrophorese und chromatographische Verfahren, insbesondere Ionenaustauschchromatographie, Ausschlusschromatographie (Gelfiltration) und Affinitätschromatographie vorgeschaltet ist; und/oder
(ii) vor der Spaltung (Schritt (a)) eine Blockierung bestimmter Monomere an der Position n innerhalb der Sequenz des Biopolymers erfolgt, so dass die Verknüpfung dieses blockierten Monomers zu dem in der Sequenz unmittelbar vorangehenden (an der Position n-1) und/oder nachfolgenden Monomeren (an der Position n+1) in Schritt (a) nicht gespalten wird; und/oder
(iii) vor der Kopplung (Schritt (b)) eine Blockierung von einem oder beiden endständigen Monomeren (Termini) des Biopolymers erfolgt, so dass die blockierten Termini in Schritt (b) nicht an das feste Trägermaterial gekoppelt werden.

4. Verfahren nach einem oder mehreren der Ansprüche 1 bis 3, wobei
(i) die Kopplung in Schritt (b) zuerst an den freien Linker erfolgt, und dann die Addukte aus Biopolymerfragmenten und Linker und/oder Addukte aus Biopolymer und Linker über eine funktionelle Gruppe des Linkers an die Ankergruppe des unlöslichen Trägermaterials gebunden werden; und/oder
(ii) der Linker eine Verbindung mit zwei gleich- oder verschiedenartigen, unter den Bedingungen des erfindungsgemäßen Verfahrens reaktiven funktionellen Gruppen ist, wovon die eine funktionelle Gruppe die Bindung an die Ankergruppe und die andere funktionelle Gruppe die Bindung an das Biopolymer oder eines oder mehrere seiner Fragmente ermöglicht; und/oder
(iii) der Linker über zwei gleich- oder verschiedenartige funktionelle Gruppen X¹ und X² ausgewählt aus -NH₂, -CN, -OH, -COOH, -COCI, -CON₃, -CHO, -NN, -SH, -SCH₃, -NNH, -CHCH₂, -NCS, -NCO, -CNO, -CNS, -SO₂Hal, -OPO₃²⁻ Oxiran und Vinylsulfon verfügt, und vorzugsweise die Formel X¹-(A)ₙ-X² aufweist (wobei A eine Aryl-, Heteroaryl-, Alkylgruppe, CH₂-Struktur, Silyl-, Etheroder Thioether-Struktur umfasst und n eine natürliche Zahl von 1 bis 20 ist, wobei A vorzugsweise einen Aminosäure-, Peptid-, Nukleosid- Nukleinsäureoder PNA-Rest zusätzlich beinhaltet oder A einem Aminosäure-, Peptid-, Nukleosid- oder Nukleinsäurerest entspricht).

5. Verfahren nach einem oder mehreren der Ansprüche 1 bis 4, wobei
(i) die in Schritt (c) erhaltene Waschlösung aufgefangen wird und die in der Waschlösung enthaltenen Biopolymerfragmente und/oder Biopolymere wiederum dem Schritt (a) und anschließend den weiteren Verfahrensschritten unterworfen werden; und/oder
(ii) für jedes Gemisch von Biopolymerfragmenten, die einer bestimmten Probe von Biopolymeren entstammen, in Schritt (e) eine bestimmte Markierung gewählt wird, so dass sich die so markierten Biopolymerfragmente dieser Probe zuordnen lassen, und wobei die Markierungen verschiedener Proben vernachlässigbar unterschiedliche oder identische Einflüsse auf das Trennverhalten der markierten Biopolymerfragmente aufweisen, bevorzugt als Markierung in Schritt (e) ein Fluoreszenzfarbstoff, eine isotopenmarkierte, chirale und/oder magnetische Verbindung an das Biopolymerfragment gebunden wird; und/oder
(iii) in Schritt (f) die markierten Biopolymerfragmente zusätzlich so getrennt werden, dass nur diejenigen Fraktionen der Charakterisierung in Schritt (g) zugeführt werden, die über unterschiedliche Intensität des aufgrund der Markierung detektierten Signals verfügen, und so eine Verminderung der in Schritt (g) einzusetzenden Fraktionen erreicht wird; und/oder vor der Trennung in Schritt (f) markierte Biopolymerfragmente aus verschiedenen Ausgangsproben vermischt werden.

6. Verfahren nach einem oder mehreren der Ansprüche 1 bis 5, wobei die Schritte (a) (Spaltung), (b) (Kopplung) und (e) (Markierung) in anderer Reihenfolge durchgeführt werden, vorzugsweise
(i) der Schritt (b) vor dem Schritt (a) durchgeführt wird, so dass die Kopplung der Biopolymere an das feste Trägermaterial vor der Spaltung erfolgt; und/oder
(ii) der Schritt (e) vor dem Schritt (a) durchgeführt wird, so dass bereits markierte Biopolymere fragmentiert werden, besonders bevorzugt die Kopplung in Schritt (b) über die Markierung erfolgt.

7. Verfahren nach einem oder mehreren der Ansprüche 1 bis 6, wobei eine Charakterisierung der in Schritt (e) getrennten, markierten Biopolymerfragmente durch spektrometrische Methoden, insbesondere durch Massenspektrometrie erfolgt.

8. Verfahren nach einem oder mehreren der Ansprüche 1 bis 7, wobei Proteine und/oder Peptide als Biopolymere und Peptide und/oder Aminosäuren und/oder deren Derivate als Biopolymerfragmente dem Verfahren unterworfen werden, und wobei in Schritt (a)
ein oder mehrere Enzyme, insbesondere ausgewählt aus Trypsin, Submaxillarus-Protease, Chymotrypsin, Staphylococcus-aureus-V8-protease, Asp-N-Protease, Pepsin, Lys-C, Glu-C, Arg-C-Proteinase, Asp-N-Endopeptidase, BNPS-Skatole, Caspasen, Chymotrypsin, Clostripain, Factor Xa, Glutamylendopeptidase, GranzymeB, Proline-Endopeptidase, Proteinase K, Staphylococcus-Peptidase I, Tehrmolysin, Thrombin, Carboxypeptidasen und eine Kombination derselben verwendet werden; oder
ein oder mehrere chemische Reagenzien ausgewählt aus Säure, insbesondere Salzsäure, TFA und Aminsäure, Phenylisothiocyanat und Bromcyan verwendet werden.

9. Verfahren nach Anspruch 8, wobei Methionin und/oder methioninhaltige Peptide nach Umsetzung mit Bromcyan durch Reaktion des entstandenen Homoserinlactons mit einer Amingruppe des Linkers oder mit einer Amingruppe auf dem unlöslichen Trägermaterial gekoppelt wird.

10. Verfahren nach Anspruch 8 oder 9, wobei
(ii) die Blockierung wie in Anspruch 3 definiert durch Umsetzung des Biopolymers mit einem Reagenz ausgewählt aus Säurehalogenide, Säureanhydriden, Aldehyde, Isocyanat-Derivate, Isothiocyanat-Derivat, Succinimid-Derivate, Imidazolyl-Carbamat-Derivate, Trauts-Reagenz-Derivate, Sulfonsäurechlorid-Derivate, Oxiran-Derivate, Imidate, Hydrazide, Sufosuccinimidyl-Derivate, Diimid-Derivate, Maleimid-Derivate, 7-Sulfobenzofurazan-Derivate, insbesondere Acetylchlorid und Citraconsäureanhydrid erfolgt; und/oder
(ii) der Linker die Formel X¹-(A)ₙ-X² aufweist, und wobei A eine Aryl-, Heteroaryl-, Alkylgruppe, CH₂-Struktur, Silyl-, Ether- oder Thioether-Struktur umfasst und n eine natürliche Zahl von 1 bis 20 ist, und X¹ und X² gleich- oder verschiedenartige funktionelle Gruppen der Formel -C(O)OR sind, wobei R aus R¹C(O)-, *ortho*-Nitrophenyl, -C(NR¹)(NHR¹), N-Oxysuccinimid und 1-Oxybenzotriazol, und R¹ aus Niederalkyl, Cycloalkyl, Aryl, Alkenyl und Alkinyl ausgewählt ist; und/oder
(iii) in Schritt (b) 1,4-Diisothiocyanatobenzol als Linker verwendet wird; und/oder
(iv) als unlösliches Trägermaterial ein Harz, insbesondere Polystyrol eingesetzt wird; und/oder
(v) die Entkopplung durch Erniedrigung des pH-Wertes erfolgt, so dass nur N-terminal gebundene Peptide entkoppelt werden und nach der Entkopplung ein ATZ (Anilinothiazolidinon) gebunden an das unlösliche Trägermaterial vorliegt; und/oder
(vi) die Markierung in Schritt (e) für die Detektion in Schritt (f) geeignete Fluoreszenzeigenschaften aufweist; und/oder
(vii) die Trennung in Schritt (f) chromatographisch, bevorzugt mittels Flüssigkeitschromatographie (LC), besonders bevorzugt mittels Hochleistungsflüssigkeitschromatographie (HPLC) an Umkehrphasen erfolgt.

11. Verfahren nach einem oder mehreren der Ansprüche 1 bis 10, das zur Bestimmung der Sequenz eines Peptids, eines Proteins, einer Nukleinsäure oder einer PNA geeignet ist.

12. Verfahren nach einem oder mehreren der Ansprüche 1 bis 11, das zur Analyse von nicht lysinhaltigen Peptiden aus fragmentierten Proteinen geeignet ist und welches die Schritte
(a) Isolieren von blockierten N-terminalen Fragmenten von Proteinen und
(b) Entkoppel aller lysinfreien Peptide umfasst, wobei die in (a) und (b) erhaltenen Fraktionen einer weiteren differentiellen Analytik zugeführt werden.

13. Verfahren nach einem oder mehreren der Ansprüche 1 bis 12, welches zur Analyse von (synthetisch) blockierten, vorzugsweise mit Citraconsäureanhydrid blockierten N-Termini von Proteinen geeignet ist, wobei nur die N-Termini von Proteinen analysiert werden.

14. Kit zur Analyse komplexer Mischungen von Biopolymeren aus einer oder mehreren Proben, umfassend wenigstens einen der folgenden Bestandteile:
(a) ein oder mehrere chemische Reagenzien oder Enzyme, wie in Anspruch 1, 8 und/oder 9 definiert, zur Spaltung der Biopolymere in Fragmente;
(b) ein oder mehrere Reagenzien zur Ausführung der Kopplung, wie in Ansprüchen 1, 2, 4 und/oder 6 beschrieben;
(c) ein oder mehrere unlösliche Trägermaterialien, wie in Ansprüchen 1 und/oder 10 definiert;
(d) einen oder mehrere Linker, wie in Ansprüchen 1 und/oder 10 definiert;
(e) ein oder mehrere Lösungsmittel zum Waschen gemäß Anspruch 1 oder 5;
(f) ein oder mehrere Reagenzien zur Ausführung der Entkopplung, wie in den Ansprüchen 1 oder 10 definiert; und
(g) ein oder mehrere Reagenzien zur kovalenten Bindung einer Markierung, wie in den Ansprüchen 1, 5 und/oder 10 definiert.

15. Kit nach Anspruch 14, wobei die Biopolymere und/oder die Fragmente ausgewählt sind, wie in Anspruch 2 definiert, und vorzugsweise die Biopolymere Proteine und die Fragmente Peptide, Aminosäuren und/oder deren Derivate sind, und/oder wobei das Kit zusätzlich einen oder mehrere Behälter mit einem oder mehreren Reagenzien zur Blockierung, wie in Anspruch 10 definiert, enthält.
